# EUROPEAN PATENT SPECIFICATION

(11) **EP 4 237 054 B1**
(45) Date of publication and mention of the grant of the patent: **07.01.2026**
(21) Application number: 21885489.1
(22) Date of filing: 29.10.2021
(51) Int. Cl.: A61M 16/06

(54) **HEADGEAR, INTERFACE AND AN ASSEMBLY**
KOPFBEDECKUNG, SCHNITTSTELLE UND ANORDNUNG
HARNAIS DE TÊTE, INTERFACE ET UN ENSEMBLE

(30) Priority: 30.10.2020 US 202063107940 P
(43) Date of publication of application: 06.09.2023
(73) Proprietor: Fisher & Paykel Healthcare Limited, East Tamaki Auckland 2013 (NZ)
(72) Inventor: HENSMAN, Sally Margaret, Auckland, 2013 (NZ); DIXON, Freya Refilwe, Auckland, 2013 (NZ); SOMERVILLE, Jemma Tamsin, Auckland, 2013 (NZ)
(74) Representative: Murgitroyd & Company
(86) International application number: PCT/IB2021/060002
(87) International publication number: WO 2022/091008

(56) References cited:
- EP-A1- 2 529 781
- WO-A1-2010/073142
- WO-A1-2010/073142
- WO-A1-2012/067523
- WO-A1-2012/140514
- WO-A1-2013/172719
- WO-A1-2014/142681
- WO-A1-2014/175753
- WO-A1-2016/075658
- WO-A1-2016/103138
- WO-A1-2019/111135
- US-A1- 2006 213 521
- US-A1- 2009 032 026
- US-B1- 6 470 886
- US-B1- 7 296 575

## Description

### FIELD

The present disclosure generally relates to components for medical systems for conveying gases to and/or from a patient. In one particular aspect, the disclosure relates to headgear, a patient interface, or an assembly of headgear and a patient interface as part of a medical system for conveying breathable gases to and/or from a patient or as part of a breathing system.

### BACKGROUND

Alternative and improved retention systems for positioning, such as fixed positioning of gas delivery interface systems, such as masks, nasal cannula or other oronasal gas delivery interface units, for a user of the interface are always being sought.

In infant applications, adhesive patches or other dermal connection systems may be used to position such gas delivery interfaces. For example, adhesive tape is applied over the tube or part of a nasal cannula to hold the cannula in place or an operable position on the infant's face. This may cause or contribute to a number of problems, such as skin reactions, skin abrasion, or breakdown when tape is repeatedly applied and removed, especially when an infant is being cycled between different types of gas therapy.

Many complex systems, including elasticised straps, buckles, tensioners, and other such retaining systems, are utilised in holding or positioning of user interfaces on the face or in preferred installation positions on a user. Stresses applied to the head of a user from various complex headgears may result in stress sores or contact abrasion.

Known devices are disclosed in WO2010/073142A1 Adjustable headgear and WO2013/172719A1 Headgear, interface and an assembly.

### SUMMARY OF THE INVENTION

The invention is defined by claim 1 and its appended dependent claims. Therefore, a system for improved ease of application or installation of such interfaces for a user, such as by the user or by a caretaker of the user are desirable. Ease of being able to cycle between different treatment therapies would also be desirable, especially also reducing the need for handling of a user's head or applying and re-applying adhesives, glues, or tapes to the face of the user for positioning of a gas delivery interface in an operational position.

**In** addition, minimizing the overall stresses applied to the head of a user is also desirable. Stresses applied to the head or face of a user, depending on where tension is exerted from such more complex headgear arrangements, can sometimes result in "snub nosing". With one or more embodiments disclosed herein, the likelihood of snub nosing preferably is reduced or eliminated.

The present disclosure aims to provide a headgear and/or an interface, or an assembly of both a headgear and an interface, which will go at least some way towards addressing the foregoing problems or which will at least provide the public with a useful choice. Moreover, the headgear can maintain the interface (e.g., a sealing interface) in an operable position so as to deliver therapy appropriately and consistently to the patient.

In a first aspect, the present disclosure provides a headgear for use with a respiratory patient interface. The headgear may comprise a backstrap portion, at least one top strap portion, a first adjustable ear loop, and a second adjustable ear loop. The first adjustable ear loop can comprise a first connection portion configured to be removably attachable to a first attachment zone of the headgear such that the first adjustable ear loop defines a first ear opening. The first attachment zone can be configured to be attachable to different portions along the first connection portion to adjust a size of the first ear opening. The second adjustable ear loop can comprise a second connection portion configured to be removably attachable to a second attachment zone of the headgear such that the second adjustable ear loop defines a second ear opening. The second attachment zone can be configured to be attachable to different portions along the second connection portion to adjust a size of the second ear opening.

In some configurations, the first and second ear openings can comprise a rounded triangular shape.

In some configurations, the first and second ear openings can be shaped and configured such that the first and second adjustable ear loops avoid the ears of a user.

In some configurations, the first and second attachment zones can comprise one of a hook material or a loop material. The first and second connection portions can comprise the other of the hook material or the loop material.

In some configurations, the at least one top strap portion can comprise a first top strap portion and a second top strap portion that can be selectively connectable to the first top strap portion.

In some configurations, the first top strap portion can extend from a first end of the backstrap portion and the second top strap portion can extend from a second end of the backstrap portion.

In some configurations, the first attachment zone can be located on the first top strap portion and/or a first protruding tab of the first top strap portion, and the second attachment zone can be located on the second top strap portion and/or a second protruding tab of the second top strap portion.

In some configurations, the first top strap portion can comprise a first protruding portion and the second top strap portion can comprise a second protruding portion.

In some configurations, the first protruding portion can comprise a third attachment zone and the second protruding portion can comprise a fourth attachment zone.

In some configurations, the headgear can further comprise a forehead securement member configured to be removably attachable to the third and fourth attachment zones.

In some configurations, the forehead securement member can comprise one of the hook material or the loop material and the third and fourth attachment zones can comprise the other one of the hook material and the loop material.

In some configurations, the first and second top strap portions can be configured to be adjustable to conform to a size of the head of the user.

In some configurations, the first top strap portion can comprise a slot configured to receive the second top strap portion.

In some configurations, the second top strap portion can comprise a plurality of adjustment indicators.

In some configurations, the plurality of adjustment indicators can correspond with a plurality of different head circumferences.

In some configurations, the first and second adjustable ear loops can be symmetrical about the backstrap portion.

In some configurations, the headgear can comprise a soft material.

In some configurations, the first and second attachment zones can comprise a hook material and the remainder of the headgear can comprise a loop material.

In some configurations, the first and second adjustable ear loops can comprise respective ear loop size indicators. The respective ear loop size indicators can correspond to a respective size of the first and second ear openings.

In some configurations, the first and second adjustable ear loops can comprise respective attachment zones.

In some configurations, the respective attachment zones can be configured to be removably attachable to the respiratory patient interface.

In some configurations, the respective attachment zones can comprise a hook material and the respiratory patient interface can comprise a loop material.

In a second aspect, the present disclosure provides a headgear for use with a respiratory patient interface. The headgear may comprise a backstrap portion, a first top strap portion, a second top strap portion, and a forehead securement member. The forehead securement member can be configured to be removably attachable to first and second attachment zones of the headgear to provide horizontal stability to the headgear in use.

In some configurations, the first top strap portion can extend from a first end of the backstrap portion and the second top strap portion can extend from a second end of the backstrap portion.

In some configurations, the first attachment zone can be located on the first top strap portion and the second attachment zone can be located on the second top strap portion.

In some configurations, the forehead securement member can further comprise a first surface that can be configured to be removably attachable to the first and second attachment zones and a second surface opposite the first surface.

In some configurations, the forehead securement member can further comprise a third attachment zone on the second surface that can be configured to be removably attachable to a removable securement member.

In some configurations, the removable securement member can be configured to couple to the respiratory patient interface.

In some configurations, the third attachment zone can comprise a hook material and the first surface can comprise a loop material.

In some configurations, the first surface can comprise a loop material, and the first and second attachment zones can comprise a hook material.

In some configurations, the first top strap portion can comprise a first protruding portion and the second top strap portion can comprise a second protruding portion.

In some configurations, the first protruding portion can comprise the first attachment zone and the second protruding portion can comprise the second attachment zone.

In some configurations, the headgear can further comprise a first adjustable ear loop extending from a first end of the backstrap and a second adjustable ear loop extending from a second end of the backstrap. The first adjustable ear loop can follow a path defining a first ear opening. The second adjustable ear loop can follow a path defining a second ear opening.

In some configurations, the first and second top strap portion can be configured to be adjustable to conform to a size of the head of the user.

In some configurations, the first top strap portion can comprise a slot configured to receive the second top strap portion.

In some configurations, the second top strap portion can comprise a plurality of adjustment indicators.

In some configurations, the plurality of adjustment indicators can correspond with a plurality of different head circumferences.

In some configurations, the headgear can comprise a soft material.

In some configurations, the first and second attachment zones can comprise a hook material and the remainder of the headgear can comprise a loop material.

In a third aspect, the present disclosure provides a patient interface assembly for delivering respiratory support to a user. The patient interface assembly can comprise a headgear and a respiratory interface. The headgear can comprise a backstrap portion, a first top strap portion, a second top strap portion, a first adjustable ear loop and a second adjustable ear loop. The first adjustable ear loop can comprise a first connection portion configured to be removably attachable to a first attachment zone of the headgear such that the first adjustable ear loop defines a first ear opening. The first attachment zone can be configured to be attachable to different portions along the first connection portion to adjust a size of the first ear opening. The second adjustable ear loop can comprise a second connection portion configured to be removably attachable to a second attachment zone of the headgear such that the second adjustable ear loop defines a second ear opening. The second attachment zone can be configured to be attachable to different portions along the second connection portion to adjust a size of the second ear opening. The respiratory patient interface can be configured to deliver respiratory support to the patient. The respiratory patient interface can be removably attachable to the headgear.

In some configurations, the respiratory patient interface can comprise a sealing patient interface or a non-sealing patient interface.

In some configurations, the respiratory patient interface can comprise a mask or a nasal cannula.

In some configurations, the first and second adjustable ear loops can comprise respective attachment zones.

In some configurations, the respective attachment zones can be configured to be removably attachable to the respiratory patient interface.

In some configurations, the respiratory patient interface can comprise a pair of connection arms. Each of the pair of connection arms can comprise respective attachment portions. The respective attachment portions can be configured to be removably attachable to the respective attachment zones of the first and second ear loops.

In some configurations, the respective attachment zones can comprise a hook material and the respective attachment portions can comprise a loop material.

In some configurations, the patient interface assembly can further comprise one or more of: a seal housing, an inspiratory conduit, an expiratory conduit, or a forehead arm.

In some configurations, the patient interface assembly can be configured to deliver a high flow therapy or a continuous positive airway pressure (CPAP) therapy.

In some configurations, the first top strap portion can comprise a first protruding portion and the second top strap portion can comprise a second protruding portion.

In some configurations, the first protruding portion can comprise a third attachment zone and the second protruding portion can comprise a fourth attachment zone.

In some configurations, the patient interface assembly can further comprise a forehead securement member configured to be removably attachable to the third and fourth attachment zones.

In some configurations, the forehead securement member can comprise one of a hook material or a loop material and the third and fourth attachment zones can comprise the other one of the hook material and the loop material.

In some configurations, the forehead securement member can further comprise one or more tacky areas comprising a tacky material configured to increase friction between the one or more tacky areas and the head of the patient.

In some configurations, the tacky material can comprise a polyurethane adhesive film, a polyurethane elastic barrier, a neoprene material, a non-stick silicone material, and/or a thermoplastic polyurethane material.

In some configurations, the headgear can further comprise one or more stiffening areas comprising a stiffening material configured to increase a strength and/or sturdiness of the one or more stiffening areas.

In some configurations, the stiffening material can comprise polypropylene, a hook material, and/or a loop material.

In some configurations, the one or more stiffening areas can be positioned on the first adjustable ear loop, the second adjustable ear loop, the first top strap portion, and/or the second top strap portion.

In some configurations, the headgear can further comprise one or more tacky areas comprising a tacky material configured to increase friction between the one or more tacky areas and the head of the patient.

In some configurations, the tacky material can comprise a polyurethane adhesive film, a polyurethane elastic barrier, a neoprene material, a non-stick silicone material, and/or a thermoplastic polyurethane material.

In some configurations, the first adjustable ear loop can comprise a first attachment section and the second adjustable ear loop can comprise a second attachment section.

In some configurations, the patient interface assembly can further comprise a chin securement member configured to be removably attachable to the first and second attachment sections.

In some configurations, the chin securement member can comprise one of a hook material or a loop material and the first and second attachment sections can comprise the other one of the hook material and the loop material.

In some configurations, the chin securement member can further comprise one or more tacky areas comprising a tacky material configured to increase friction between the one or more tacky areas and the head of the patient.

In some configurations, the tacky material can comprise a polyurethane adhesive film, a polyurethane elastic barrier, a neoprene material, a non-stick silicone material, and/or a thermoplastic polyurethane material.

In some configurations, the chin securement member can further comprise a slit positioned on the chin securement member such that the slit aligns with the chin of the patient in use.

Further aspects and advantages of certain embodiments of the disclosure will become apparent from the ensuing description, which is given by way of example only.

### BRIEF DESCRIPTION OF THE DRAWINGS

Various embodiments are depicted in the accompanying drawings for illustrative purposes, and should in no way be interpreted as limiting the scope of the embodiments. Furthermore, various features of different disclosed embodiments can be combined to form additional embodiments, which are part of this disclosure.
Figure 1 is a front view of a user wearing a headgear connected to a respiratory patient interface according to certain aspects of the present disclosure.
Figure 2 is a side view of the user wearing the headgear connected to the respiratory patient interface shown in Figure 1.
Figure 3 is a top view of an outward-facing surface of a headgear according to certain aspects of the present disclosure.
Figure 4 is a top view of a patient-contacting surface of the headgear shown in Figure 3.
Figure 5 is a front view of a user wearing a headgear connected to a respiratory patient interface according to certain aspects of the present disclosure.
Figure 6 is a side view of the user wearing the headgear connected to the respiratory patient interface shown in Figure 5.
Figures 7-9 illustrate various perspective views of the user wearing the headgear connected to the respiratory patient interface shown in Figure 5.
Figures 10 is a top view of a forehead securement member according to certain aspects of the present disclosure.
Figure 11 is a front view of a user wearing a headgear connected to a respiratory patient interface according to certain aspects of the present disclosure.
Figure 12 is a front view of a user wearing a headgear connected to a respiratory patient interface according to certain aspects of the present disclosure.
Figure 13 is a front view of a headgear according to certain aspects of the present disclosure.
Figure 14 is a perspective view of the headgear shown in Figure 13.
Figure 15 is a front view of a user wearing the headgear shown in Figure 13.
Figure 16 is a top view of an outward-facing surface of a headgear according to certain aspects of the present disclosure.
Figure 17 is a top view of a patient-contacting surface the headgear shown in Figure 16.
Figure 18 is a top view of an outward-facing surface of a forehead securement member according to certain aspects of the present disclosure.
Figure 19 is a top view of a patient-contacting surface the forehead securement member shown in Figure 18.
Figure 20 is a front view of a user wearing a headgear connected to a respiratory patient interface according to certain aspects of the present disclosure.
Figure 21 is a side view of a user wearing the headgear connected to the respiratory patient interface shown in Figure 20.
Figure 22 is a perspective view of a user wearing a headgear connected to a respiratory patient interface according to certain aspects of the present disclosure.
Figure 23 is a top view of a patient-contacting surface of a chin securement member according to certain aspects of the present disclosure.

### DETAILED DESCRIPTION

The present application relates to headgears for securing a user interface to a user, as well as interface systems including such headgear and methods of making and using such headgear. The headgear can be easily and conveniently removable from, or installable on, the head of the user. Advantageously, the headgear can allow for minimal handling of the user or user's head during installation of the headgear or installation of a user interface to the user to an operational position. Further, one or more embodiments of the present disclosure provides for improved security of maintaining the position of the user interface on the user (e.g., prongs remaining in position in the nares of a user's nose) from the inter-connection of the respiratory user interface with the headgear.

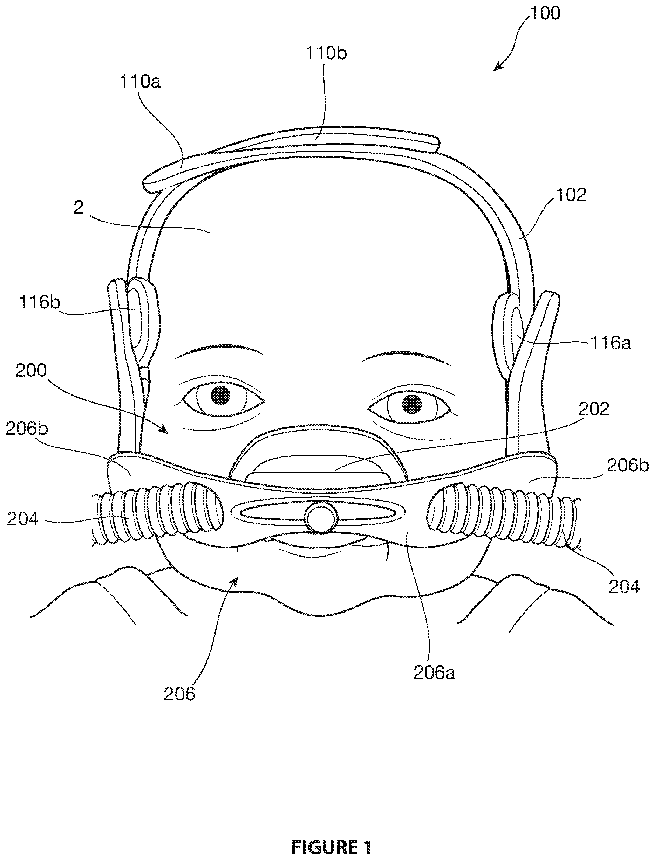

Figures 1-2 illustrate a headgear 100 releasably coupled to a user interface 200 and attached to the head 2 of a user. The headgear 100 can be easily and conveniently removable from, or installable on, the head 2 of a user. The headgear 100 can comprise a body 102 configured to engage with the head 2 of the user. The body 102 can extend generally about a rear region 4 of a user's head 2, generally about an upper region 6 of the user's head 2, and generally about an ear 8 or both ears 8 of the user.

The user interface 200 can be configured to deliver breathing gases to the airways of the user. The user interface 200 can be suitable for various forms of respiratory support. For example, the user interface 200 can be suitable for delivering high flow therapy and/or continuous positive airway pressure (CPAP) therapy. The headgear 100 may be suitable for use in these types of systems. For example, the headgear 100 may be suitable for maintaining a seal of the user interface 200 during CPAP therapy. The user interface 200 may be a sealing or non-sealing mask or cannula, or other suitable interface. The user interface 200 can include a support portion, such as a frame 206, and a user-interfacing portion, such as a cushion module 202 or a nasal cannula module, that is connected to the frame 206. The frame 206 can be in the form of an elongate, laterally-extending element or assembly, which includes a central portion 206a and a pair of side arms 206b on each side of the central portion 206a. The user-interfacing portion 202 can be supported by or connected to the central portion 206a of the frame 206. However, the frame 206 can be of any other suitable structure for supporting the user-interfacing portion 202 and connecting to the headgear 100. In some arrangements, the frame 206 can be omitted and the user-interfacing portion 202 can be coupled directly to the headgear 100.

One or both of the cushion module 202 and the nasal cannula module can include a connection portion and a user-engaging portion, which can be a cushion (e.g., seal) in the cushion module or nasal elements (e.g., prongs or pillows) in the nasal cannula module. The connection portion can be configured to directly or indirectly connect to the frame 206. In some arrangements, the connection portion can be more rigid than the user-engaging portion. In some configurations, the connection portion can be or comprise a housing or a clip. Such an arrangement allows for convenient and reliable interchangeability among a mask, a cannula, or other suitable interface. In other arrangements, the user-engaging portion can be directly connected to the frame 206 or other support portion of the user interface 200. The user interface 200 can include one or more conduits 204 in fluid communication with the user- interfacing portion. For example, the one or more conduits 204 can comprise an inspiratory conduit and/or an expiratory conduit. The conduits 204 can be directly connected to the user- interfacing portion (e.g., cushion module 202) or can be indirectly connected to the user- interface portion, such as through the frame 206. Examples of headgear and interface having similar constructions in a general sense and used in similar applications are disclosed in WO2012/067523 and WO2013/172719.

The headgear 100 can be configured to be ergonomic and to provide reduced or minimal coverage to expose as much of the patient's (e.g., an infant) face as possible or practical when the patient is receiving therapy. This configuration provides comfort to the patient because the headgear 100 has minimal straps and contact regions with the patient's face. It also provides comfort to the caregiver(s) and/or parent(s) since they can see the patient's face. This configuration can also improve the clinicians' ability to observe the patient. For example, the clinicians can inspect potential damage sites.

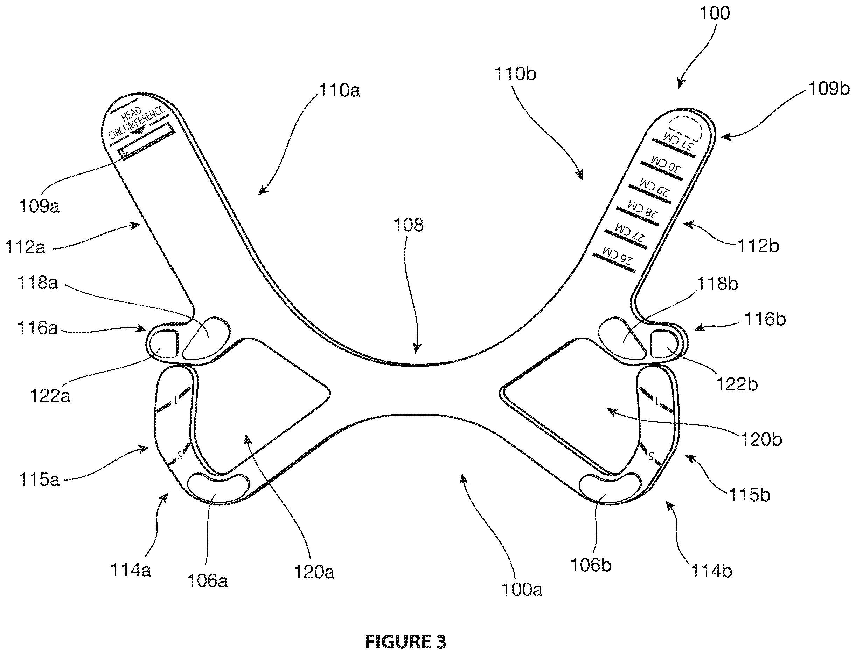

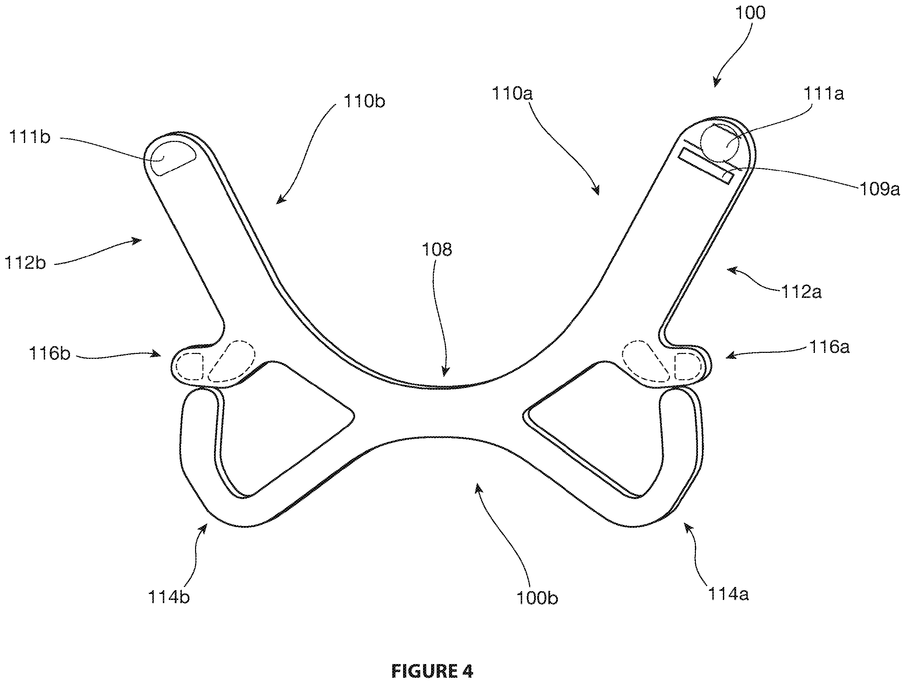

The body 102 and/or other portions of the headgear 100 can comprise a soft material. The soft material can be gentle on the patient's skin and reduce the potential for skin damage from abrasion. The soft material of the headgear 100 can conform to the user's head 2, which can help when fitting the headgear 100 onto the patient. Furthermore, the headgear 100 can have, for example, an inner, patient-contacting side 100b, as shown in Figure 4, and an outward-facing side 100a, as shown in Figure 3. One or both external surfaces can comprise a loop material. The loop material can be beneficial for many of the adjustment mechanisms disclosed herein. For example, one or both of first and second top straps 110a, 110b, which are further described below in relation to Figures 3 and 4, can comprise a corresponding hook portion and can therefore attach anywhere along the other one of the first and second top straps 110a, 110b that has an external loop surface.

The headgear 100 can couple to the user interface 200. For example, the headgear 100 can releasably couple to the user interface 200. In the illustrated arrangement, the headgear 100 selectively couples to the user interface 200 via a releasable connection system 104. The releasable connection system 104 can be a two-part releasable connector system. For example, a headgear connector part or connection zone 106 can be on or about a region of the body 102 extending generally about the ear 8 or ears 8 of the user, or can be provided on or about a region of the body 102 extending generally at or in front of the ear 8 or ears 8 of the user (as shown in Figures 1-2). An interface connector part or connection zone 206c may be provided by, or on, a region of (or in attachment or connection with) the user interface 200. For example, as discussed above, the frame 206 of the user interface 200 can comprise one or more side arms or connection arms 206b. Each of the one or more connection arms 206b can include an interface connector part or connection zone 206c on one or both surfaces of the one or more connections arms 206b.

It will be appreciated that various alternative releasable fasteners or releasable type connection systems can be utilized with this invention. In one configuration, the releasable connection system 104 can be a hook and loop fastener system. For example, the headgear connector part or connection zone 106 can be one of a hook or a loop and the interface connector part or connection zone 206a can be the other of the hook or the loop. The hook and loop fastener system can provide removable attachment of the user interface 200 and the interface 200 may be swapped out between different types of interfaces or different sizes of interfaces whilst the headgear 100 remains in place. This configuration allows for cycling between a sealing mask and a cannula during CPAP therapy. As discussed above, the user interface 200 may be a sealing or non-sealing mask or cannula, or other suitable interface.

In some configurations, a mushroom-type hook and loop fastener system may be used. In some configurations, the releasable connection system 104 can include adhesive material. For example, the headgear and interface connector parts or connection zones 106, 206c can include adhesive material that are connectable to, or receivable of, one another. The headgear connector part or connection zone 106 may include an adhesive section, or one part of a two-part adhesive connection system; and the interface connector part or connection zone 206c may be a receiver of the first adhesive section, or the second part of a two-part adhesive system. Such adhesives and connector parts or connection zones 106, 206c can be provided in a form so as to be releasable from each other. In some configurations, one of the headgear or interface connector part or connection zone 106, 206c may include an L- shaped retaining hook, which can be inserted into a slot provided in the other one of the headgear or interface connector part or connection zone 106, 206c. In some configurations, one of the headgear or interface connector part or connection zone 106, 206c may include a dome, which is shaped to fit into one or more slots in the other one of the headgear or interface connector part or connection zone 106, 206c. The slots may be keyhole-shaped, to prevent accidental removal of the dome from the slot. In some configurations, the releasable connection system 104 can include a snap-fit, push-fit, or an interference-fit connection system.

Advantageously, the releasable connection system 104 utilized can be of a substantially similar profile, or substantially similar to the profile of the body 102. As shown in Figures 1-2, the headgear 100 and/or the releasable connection system 104 can have a generally flat or low profile such that it is not bulky on the patient's face. This beneficially allows for increased comfort and minimizing pressure sores, while minimizing the visual impact or obstructions that other headgear of more raised exterior profiles may have or show. Part of the challenge in ensuring compliance with gas delivery systems by infant users is the visual impact that more obtrusive headgear or other systems have on the caregivers of the user or parents or guardians of infants.

For example, some prior art headgear systems are relatively obtrusive both in their overall size and visual impact. Such obtrusiveness and visual impact can be distressing to the caregivers or parents of infants undergoing treatment supplied by a user interface. It is therefore an aim of one or more of the disclosed embodiments to provide a headgear system which aids in minimizing obtrusiveness or visual impact to the caregivers of infants wearing such headgear, and/or which provides for a headgear and user interface set-up which is easier and less complex that those systems provided previously.

In some configurations, advantageously, the releasable connection system 104 does not utilize pulley strap systems or buckles. Pulley or buckle type systems can impact a user's face, particularly an infant's face, such as by pressure sores. For example, application of a connection or retention system too tightly on a user's face may contribute to so-called "snub nosing", such as when forces are applied to the nose, septum or philtrum. Such "snub nosing" is to be substantially avoided or reduced. This is uncomfortable for the user (particularly infant users), and distressing for the caregiver or parents of the infant. Therefore, the headgear connector part or connection zone 106 of the headgear 100 can be, for example, of a substantially non-elastic part, thereby preventing or minimizing rotation and/or pivoting of the headgear connector part or connection zone 106, which may otherwise compromise the position of the user interface 200 and capacity to deliver effective therapy. Thus, the user interface 200 can be generally maintained in an operational position (e.g., by maintaining the prongs of a nasal cannula in the nares of a user's nose)

In some configurations, the releasable connection system 104 can include a quick fit or quick release system or method for ease of installation or removal of the user interface 200 from the user and/or the headgear 100. Specifically, preferred embodiments reduce the likelihood of the need for application of tension during installation of the user interface 200 to a user in combination with the headgear 100. Instead, the user interface 200 can be placed into its required user interfacing position, the interface connector part or connection zone 206c of the user interface 200 can be quickly and relatively easily located upon the headgear 100. In this manner, a caregiver or installer of the user interface 200 can, with ease and minimal need for additional help, install and locate the user interface 200 in its required or desirable operational position.

One or more embodiments of the present disclosure beneficially reduce the need for application of adhesive, or adhesive tape, to a user's skin for the installation and placement of a user interface 200 into an operational position. Adhesive tapes or other dermal adhesive patches, particularly for infants, can create problems. Problems include, but are not limited to, skin irritation from adhesive chemicals (or adhesive removal chemicals, such as solvents) or tape materials (e.g. due to skin sensitivities), damage to user skin due to repeated application and removal of dermal patches or tapes for positioning or re-positioning of the interface 200 for the user. Re-positioning may be required or adjustments may be needed where treatment therapies are being cycled (i.e., changed from one type of treatment to another, and then back again). Advantageously, therefore, one or more disclosed embodiments provides for a system of positioning or locating of a user interface 200 on a user, yet reducing the likelihood of the problems associated with adhesive tapes attached to the user's skin.

Figures 3-4 illustrate the headgear 100 laid flat. The headgear 100 can include the inner or inward-facing, patient-contacting surface 100b (Figure 4) and the outward-facing surface 100a (Figure 3) opposite the patient-contacting surface 100b. The headgear 100 can be or comprise a single integral component (e.g., body 102), as shown in Figures 3-4, or can include multiple connected or connectable components or portions. Advantageously, the illustrated headgear 100 including a single integral component or body 102 is easy to manufacture and is easy for a caretaker and/or clinician to install the headgear 100 on a patient.

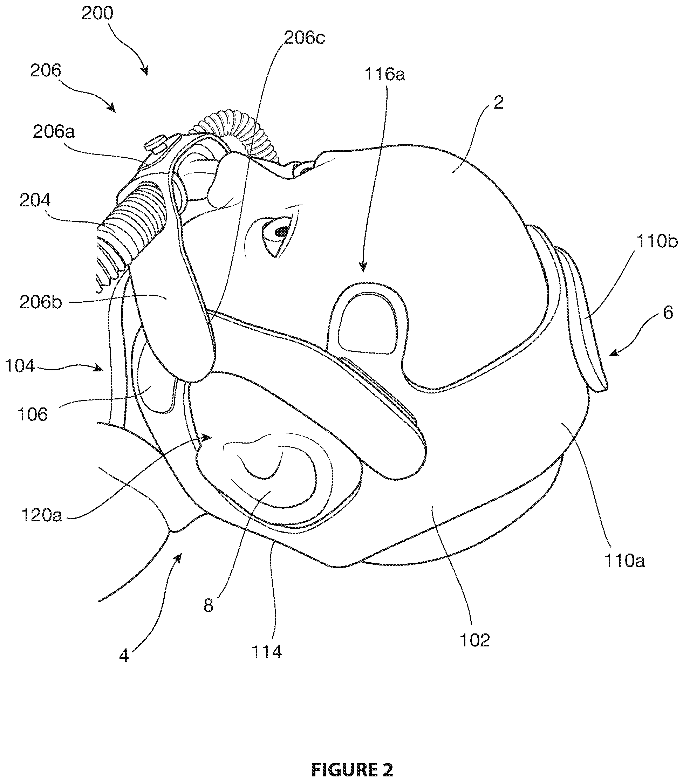

The headgear 100 can comprise a backstrap portion 108, at least one top strap 110 (e.g., first and second top straps 110a, 110b), and first and second adjustable ear loops 114a, 114b. The backstrap portion 108 can extend generally about the rear region 4 of the user's head 2 (Figure 2) in use. The first and second top straps 110a, 110b can each include a top strap portion 112a, 112b and a protruding tab portion 116a, 116b. The first and second top strap portions 112a, 112b can extend generally about the upper region 6 of the user's head 2 in use (Figures 1 and 2) and can selectively connect to one another, as further described below in relation to Figures 3 and 4. The first and second protruding tab portions 116a, 116b can extend along the side of the patient's head 2 (Figures 1 and 2) in use. For example, the protruding tab portions 116a, 116b can generally or substantially align with the patient's forehead when the headgear 100 is properly fitted to the patient.

In some configurations, the at least one top strap 110 can comprise a single top strap. In some configurations, the single top strap can be removable and configured to connect to one or more portions of the headgear 100, such as the backstrap portion 108, at least one of the first and second adjustable ear loops 114a, 114b, or any other suitable portion of the headgear 100. In some configurations, the single top strap can be integral with or permanently attached to the headgear 100. For example, one end of the single top strap could be integral with or permanently connected to the headgear 100 and the other end could be releasably connectable to the headgear 100. Alternatively, both ends of the single top strap could be integral with or permanently connected to the headgear 100.

The adjustable ear loops 114a, 114b, can extend generally about the ears 8 of the user and forming first and second ear openings 120a, 120b about the ears 8 in use. The adjustable ear loops 114a, 114b may partially surround or encircle each, or both, ears 8, or may wholly surround or encircle the ear 8 or both ears 8. The figures illustrate one configuration where the ears 8 are wholly encircled by the first and second adjustable ear loops 114a, 114b. It will be appreciated that alternative shapes or forms are contemplated.

The headgear 100 can be size-adjustable. Adjustment allows for fitment to a particular user's head 2, such as for improved anatomical adjustment or differing head size. The headgear 100 can be adjustable for varying the distance the body 102 extends between the backstrap portion 108 and the first and second adjustable ear loops 114a, 114b. Additionally, or alternatively, the headgear 100 can be adjustable for varying the distance the body 102 extends between the first and second top strap portions 112a, 112b and the first and second adjustable ear loops 114a, 114b. Additionally, or alternatively, the first and second adjustable ear loops 114a, 114b can be adjustable. For example, an adjustment can be made to the size of the ear openings 120a, 120b about an ear 8 or each ear 8 of the user. That is, the ear loop 114a, 114b encircling partially or wholly the ear 8 or ears 8 of a user can be made smaller or larger, depending on the dimensions of the head 2 of the user.

As shown in Figures 3-4, the top strap portions 112a, 112b can be adjustable and have complementary ones of a two-part releasable connection system. In some configurations, the two-part releasable connection system can be or include a hook and loop system, any of the releasable connection systems described in this application, or any other suitable releasable connection. In the illustrated arrangement, the first top strap portion 112a can include a slit 109a that is configured to receive the second top strap portion 112b, or vice versa. As shown in Figure 3, the outward-facing surface 100a of the second top strap portion 112b can include one or more indicators 109b. The one or more indicators 109b can include visual indicators and/or tactile indicators. The one or more indicators 109b can indicate a size of at least a portion of the headgear 100 formed when the first top strap portion 112a and the second top strap portion 112b are coupled. For example, the size can relate to a length of the top strap 110 and/or a circumference of the headgear 100. The size indicated by the indicators 109b can be relative sizing, such as two or more of extra-small (XS), small (S), medium (M), large (L), and extra-large (XL), for example. In the illustrated arrangement, the size indicated by the indicators 109b can indicate an absolute size. In particular, the illustrated indicators 109b indicate a head circumference, which can assist a clinician or caretaker in fitting and/or adjusting the headgear 100 on the patient's head.

The one or more indicators 109b can allow for a coarse adjustment of the headgear 100 to an estimated head circumference measurement before it is fitted to the patient. The caretaker or clinician can make finer adjustments to the headgear 100 once it is on the patient. Advantageously, this saves time and allows for less handling of the headgear 100 when it is on a patient, such as an infant patient. In use, the caretaker can insert the second top strap portion 112b into the slit 109a of the first top strap portion 112b until the slit 109a aligns with the appropriate indicator of the one or more indicators 109b. The caretaker can secure the second top strap portion 112b to the first top strap portion 112a via the two-part releasable connection system. For example, as shown in Figure 4, the second top strap portion 112b can include a second connecting section 111b on the patient-contacting surface 100b of the second top strap portion 112b. The second connecting section 111b can include a hook material configured to engage with the loop material on the outward-facing surface 100a of the first top strap portion 112a. The caretaker can also secure the first top strap portion 112a to the second top strap portion 112b. For example, as shown in Figure 4, the first top strap portion 112a can include a first connecting section 111a on the patient-contacting surface 100b of the first top strap portion 112a. The first connecting section 111a can include a hook material configured to engage with the loop material on the outward-facing surface 100a of the second top strap portion 112b.

In some configurations, the headgear 100 can include a buckle arrangement such that one of the top strap portions 112a, 112b includes a buckle that can be configured to receive the other one of the top strap portions 112a, 112b. For example, the first top strap portion 112a can include a buckle that is configured to receive the second top strap portion 112b. The second top strap portion 112b can be passed through the buckle and secured on the first top strap portion 112a with the use of a releasable connection system, such as the hook and loop type system described above. In another alternative arrangement, each top strap portion 112a, 112b can be passed through a buckle, folded over onto themselves and secured via a releasable connection system. In yet another alternative arrangement, each top strap portion 112a, 112b can include a part of a releasable snap buckle and one or both of the top strap portions 112a, 112b can be size adjustable relative to its respective buckle part. Any of the releasable connection systems described in this application can also be used. Any of the alternative adjustment arrangements can incorporate size indicators, such as those described herein, to assist a caregiver in adjusting a size of the headgear 100.

In some configurations, the backstrap portion 108 can include one or more adjustable straps (not shown). The one or more adjustable straps can include similar adjustment strap systems as described above in relation to the top strap portions 112a, 112b. However, the present inventors have determined that a suitable range of adjustment can be provided by the illustrated headgear 100, which advantageously is provided by a one-piece, integrated body 102 for simplicity, ease of use and/or ease of manufacture.

As shown in Figures 3-4, the headgear 100 can include the first and second adjustable ear loops 114a 114b configured to avoid contacting, pressing against, or rubbing the user's ears in use. The first and second adjustable ear loops 114a 114b can form respective first and second ear openings 120a, 120b configured to receive the ears of the user. The first and second adjustable ear loops 114a 114b can be symmetrical about the backstrap portion 108. This symmetry can assist with uniformity of the headgear 100 and tensioning/positioning of the first and second adjustable ear loops 114a 114b on the patient. Referring to Figure 2, the adjustable ear loops 114a, 114b may be configured to extend about the ears 8 of the user and may partially surround or encircle each, or both, ears 8, or may wholly surround or encircle the ear 8 or both ears 8. The adjustable ear loops 114a, 114b can form a rounded triangular shape around the user's ear 8 or ears 8.

As shown in Figures 2-4, the adjustable ear loops 114a, 114b may be configured to be adjustable such that the ear loops 114a, 114b may be changed to fit a variety of different head and ear sizes. To be adjustable, the adjustable ear loops 114a, 114b may include any of the releasable connection systems described herein. For example, the patient- contacting surface 100b of the first and second adjustable ear loops 114a, 114b may include a loop material configured to releasably attach to one or more attachment zones of the headgear 100. The protruding tab portions 116a, 116b of the first and second top straps 110a, 110b can, for example, include respective first and second attachment zones 118a, 118b. The first and second attachment zones 118a, 118b can be located on the portion of the protruding tab portions116a, 116b adjacent the first and second top strap portions 112a, 112b. Alternatively, the first and second attachment zones 118a, 118b can be located anywhere on the first and second top straps 110a, 110b or the backstrap portion 108. The first and second attachment zones 118a, 118b can include corresponding hook material configured to engage with the patient-contacting surface 100b of the first and second adjustable ear loops 114a, 114b. The sizes of the first and second ear openings 120a, 120b formed by the first and second adjustable ear loops 114a, 114b can be made larger or smaller depending on where the attachments zones 118a, 118b are secured to on the ear loops 114a, 114b. Although a hook and loop connection system is described, the first and second adjustable ear loops 114a, 114b and the first and second attachment zones 118a, 118b can include any of the releasable connection systems described herein.

The adjustable ear loops 114a, 114b may include one or more indicators 115a, 115b on the outward-facing surface 100a of the ear loops 114a, 114b. The one or more indicators 115a, 115b may include visual indicators and/or tactile indicators. The one or more indicators 115a, 115b may correlate with a size of the first and second ear openings 120a, 120b. The size indicated by the one or more indicators 115a, 115b can be relative sizing, such as two or more of extra-small (XS), small (S), medium (M), large (L), and extra-large (XL), for example. In the illustrated arrangement, the one or more indicators 115a, 115b include a small (S) indicator and a large (L) indicator, which can assist a clinician or caretaker in fitting and/or adjusting the headgear 100 on the patient's head. Moreover, the one or more indicators 115a, 115b can assist the clinician or caretaker to achieve at least a preliminary fit prior to putting the headgear 100 on the patient such that little to no adjustments may be needed once the headgear 100 is on the patient. The one or more indicators 115a, 115b can also allow for uniform fitting of the adjustable ear loops 114a, 114b on each of the patient's ears. In some configurations, the indicators 115a, 115b can indicate a head circumference. In some configurations, the size indicated by the indicators 115a, 115b can indicate an absolute size. When the adjustable ear loop 114a, 114b are attached to the respective attachment zones 118a, 118b such that the large size indicator aligns with the respective protruding tab portion 116a, 116b, as shown in Figure 2, the ear opening 120a, 120b will be larger than if the small size indicator aligns with the respective protruding tab portion 116a, 116b. Advantageously, this allows the adjustable ear loops 114a, 114b to avoid the user's ears in use to inhibit or avoid damage to the ears, which may occur if the adjustable ear loops 114a, 114b overlay or otherwise contact the ears. Furthermore, the adjustability of the first and second adjustable ear loops 114a, 114b, and/or the first and second top strap portions 112a, 112b, allows a size of headgear 100 to fit a large range of patients or to stay on a single patient as the patient grows. A patient's growth can be quite a rapid change in head size for some patient populations, e.g., between 20-30 weeks of age.

The first and second adjustable ear loops 114a, 114b can include respective headgear connection zones 106a, 106b on the outward-facing surface 100a of the adjustable ear loops 114a, 114b. The first and second headgear connection zones 106a, 106b can provide areas for connecting a portion of an interface 200 (e.g., the one or more connection arms 206) to the headgear 100. The headgear connection zones 106a, 106b can be located on lower and/or forward portions of the ear loops 114a, 114b in use. The headgear connection zones 106a, 106b can be located on intermediate portions of the ear loops 114a, 114b between a fixed end and a free or adjustable end. In the illustrated arrangement, the fixed end is connected to or adjacent the backstrap portion 108 and the free end is configured to be attached to or adjacent the top straps 110a, 110b. However, this arrangement could also be reversed such that the fixed end of each ear loop 114a, 114b is connected to or adjacent the top straps 110a, 110b and the free end is configured to be attached to or adjacent the backstrap portion 108.

The first and second headgear connection zones 106a, 106b may include portions or parts of a fastener or connection system. For example, the first and second headgear connection zones 106a, 106b may include one part of a two-part connection system such as either the hook or loop components of a hook and look fastener system. In some configurations, the first and second headgear connection zones 106a, 106b may include a receptive surface for connection of an adhesive fastener system, such as a smooth plastic surface. In some configurations, the first and second headgear connection zones 106a, 106b may include a clip component for connecting with a clip component of the patient interface 200. The two-part connection system can include any of the releasable connection systems described herein.

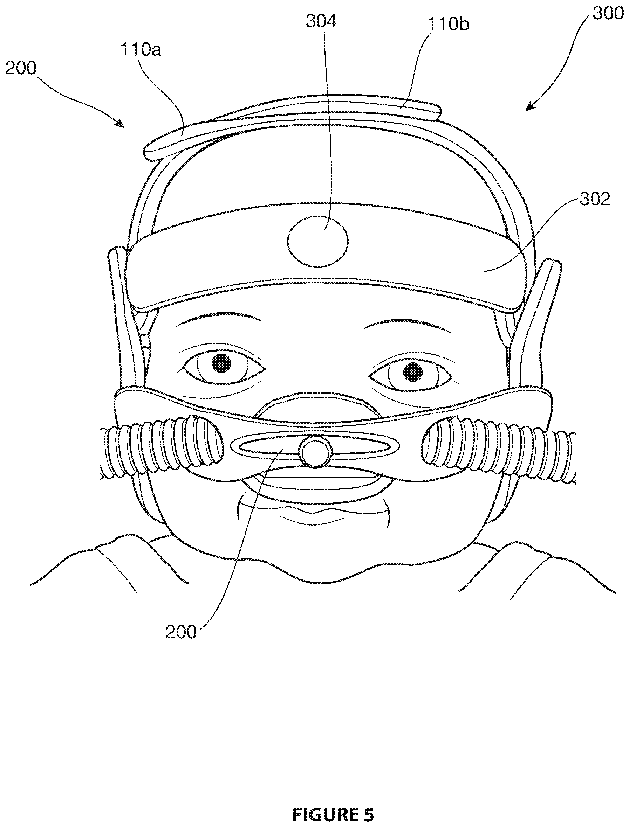

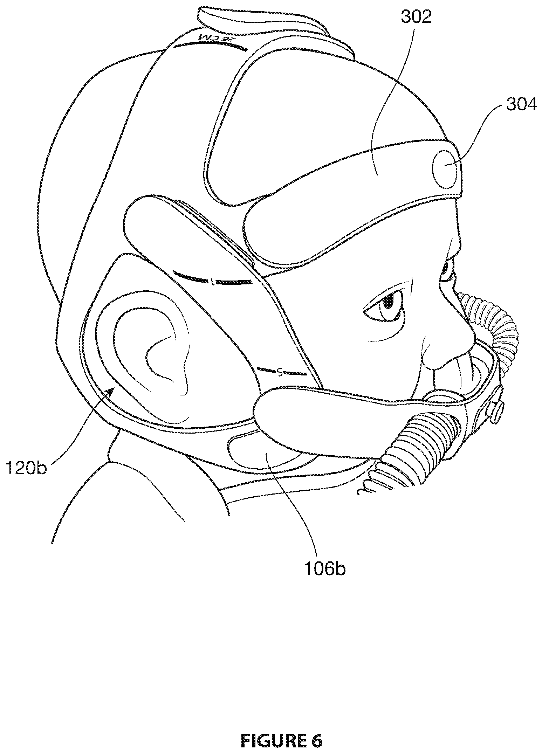

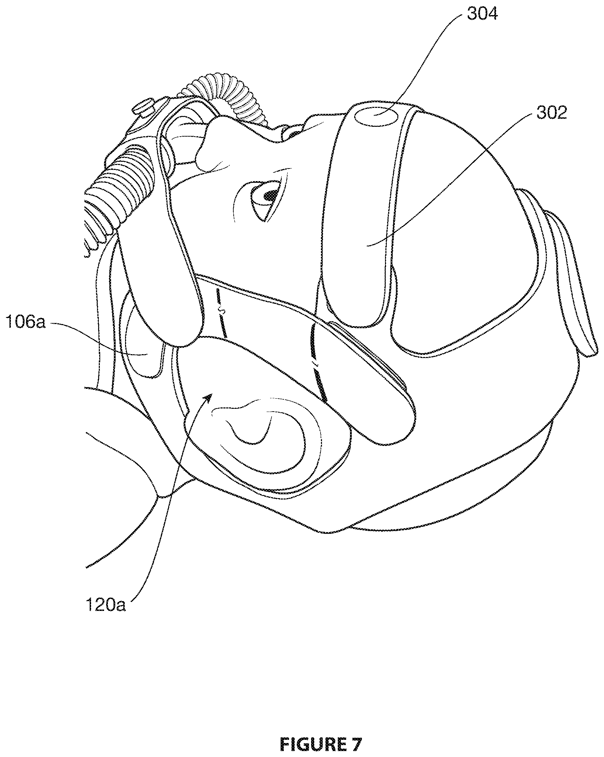

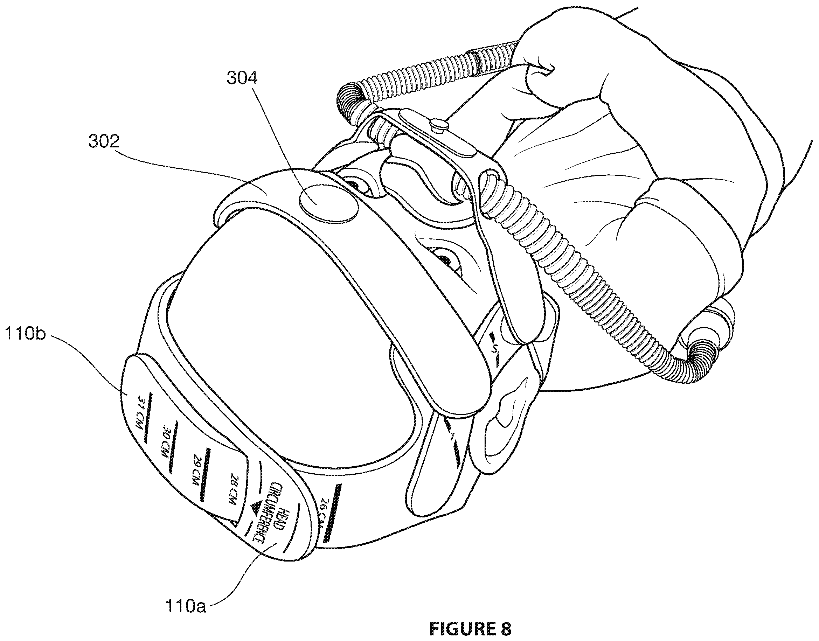

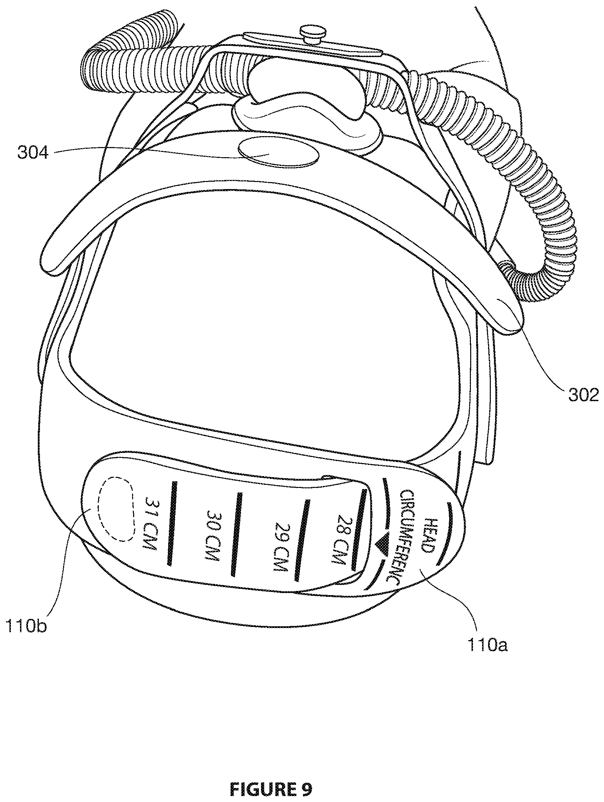

Figures 5-9 illustrate a headgear assembly 300 connected to an interface 200. The headgear assembly 300 can include the headgear 100 and a removable forehead securement member 302 configured to provide additional horizontal stability to the headgear 100 in use. The removable forehead securement member 302 can comprise a thin, elongate strap that can extend horizontally or substantially horizontally. The material and construction of the removable forehead securement member 302 can be the same as or similar to the body 102 of the headgear 100. The forehead securement member 302 preferably is removable from the body 102 or a remainder of the headgear 100; however, in alternative configurations the forehead securement member 302 can be integral with or permanently attached to the headgear 100. For example, one end of the forehead securement member 302 could be integral with or permanently connected to the headgear 100 and the other end could be releasably connectable to the headgear 100. Alternatively, both ends of the forehead securement member 302 could be integral with or permanently connected to the headgear 100.

The removable forehead securement member 302 can include a patient-contacting surface (not shown) and an outward-facing surface 302a opposite the patient-contacting surface. The patient-contacting surface of the removable forehead securement member 302 can include a first portion of a two-part releasable connection system. The protruding tab portions 116a, 116b of the headgear 100 can include third and fourth connection zones 122a, 122b on the outward-facing surface 100a of the protruding tab portions 116a, 116b (Figure 3). The third and fourth connection zones 122a, 122b can include a second portion of the two-part releasable connection system. For example, the patient-contacting surface of the removable forehead securement member 302 can include a loop material that corresponds with a hook material of the third and fourth connection zones 122a, 122b such that the third and fourth connection zones 122a, 122b are attachable to any portion of the patient-contacting surface of the removable forehead securement member 302. The two-part releasable connections system can comprise any of the releasable connection systems described herein.

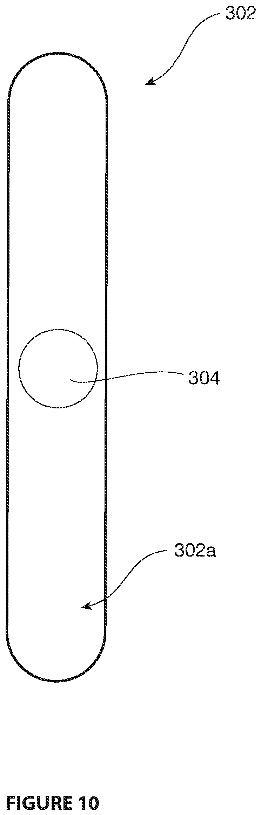

As illustrated in Figures 5-9, the removable forehead securement member 302 can be used on its own as a forehead strap to provide additional stability to the headgear assembly 300. However, in other arrangements, the forehead securement member 302 can be configured for connection to other portions of the interface assembly, such as the interface 200 or another portion of the headgear 300. As illustrated in Figure 10, among others, the removable forehead securement member 302 can include a connection zone 304. The connection zone 304 is configured to allow for another component to be connected to the connection zone 304. For example, the connection zone 304 can be positioned in the middle or approximately the middle of the outward-facing surface of the removable forehead securement member 302. The connection zone 304 can comprise one part of any of the two- part releasable connection systems disclosed herein, or can be another suitable releasable connection.

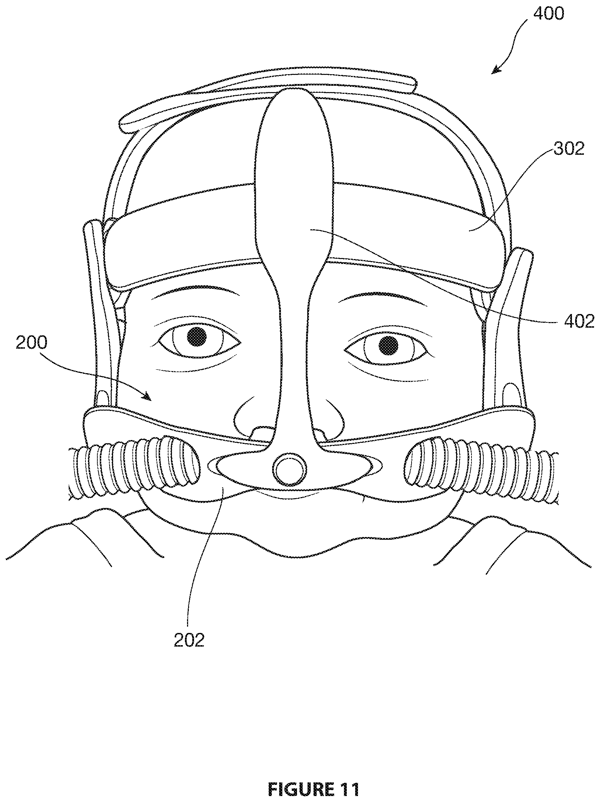

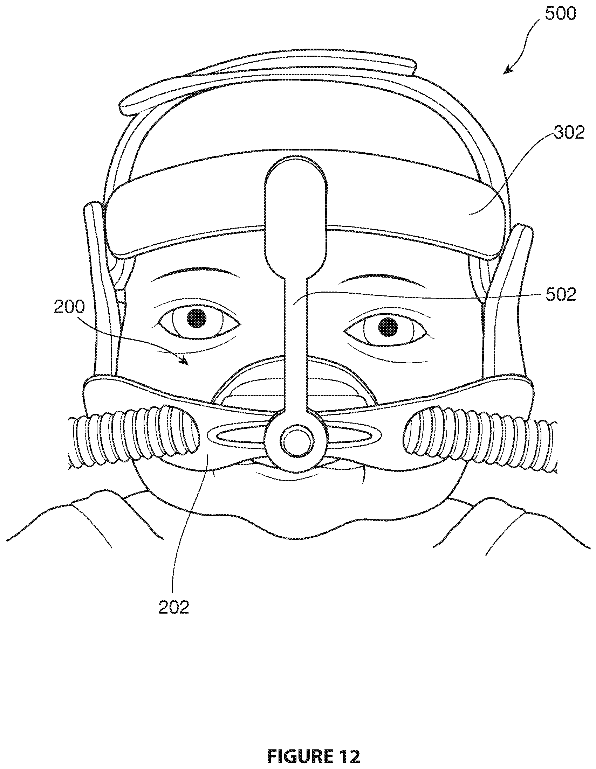

As shown in Figures 11 and 12, the removable forehead securement member 302 may be a component of other configurations of headgear assemblies 400, 500 that include a removable securement member 402, 502. In some configurations, the removable securement member 402, 502 can comprise a forehead arm 402, 502. The connection zone 403 can include a hook material that corresponds with a loop material of the removable securement member 402, 502. The removable securement member 402, 502 may extend vertically or substantially vertically. However, the removable securement member 402, 502 may be positioned in any suitable orientation. The removable securement member 402, 502 may be detachably secured to the forehead securement member 302 and the interface 200. For example, the interface 200 can include the seal housing 202 and the forehead securement member 302 may be detachably secured to the seal housing 202 of the interface 200. The interface 200 can be, for example, a nasal cannula (Figure 11) or a sealing mask (Figure 12). The removable securement member 402, 502 can provide vertical support and stability to the interface 200 to ensure a sufficient seal in some situations.

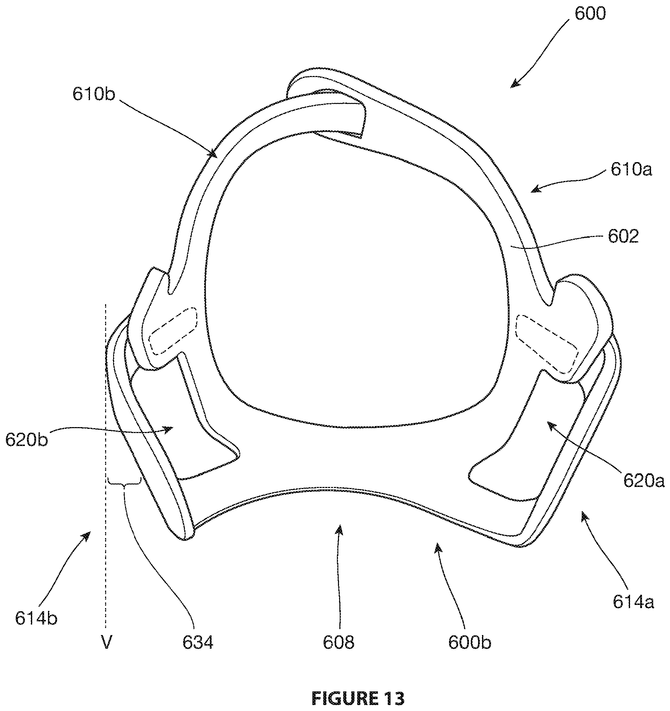

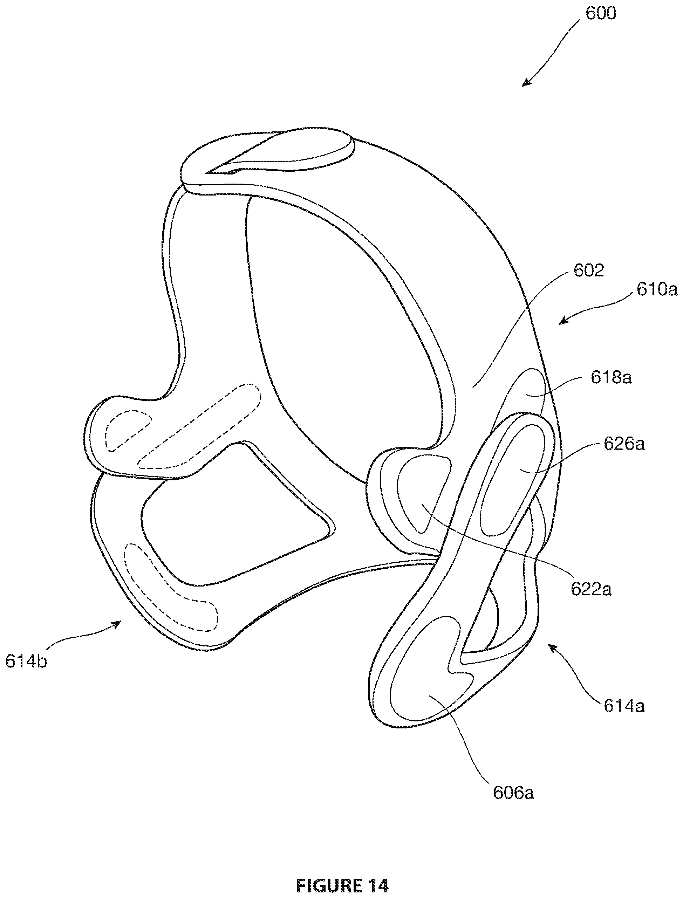

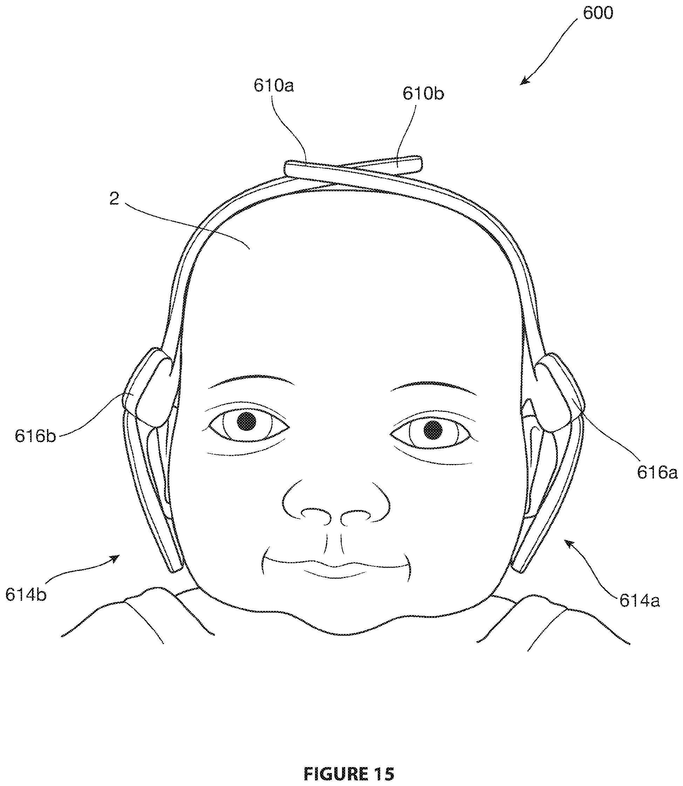

Another configuration of a headgear is illustrated in Figures 13-17. Figures 13-17 show a configuration of a headgear 600 that is similar to the headgear 100 described with reference to Figures 1-9 and can have similar features. Reference numerals of the same, substantially the same, or corresponding features may share the same last two digits. Any component disclosed in any configuration in this specification can be used in any other configuration.

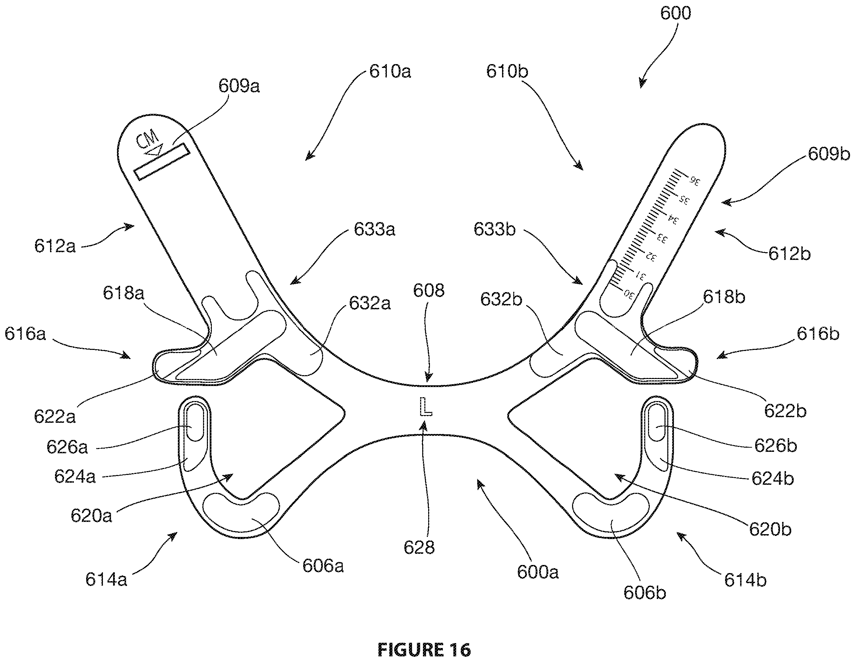

Figures 13-15 illustrate the headgear 600 formed to receive a patient's head 2 and being worn on a patient. Figures 16-17 illustrate the headgear 600 laid flat. As shown in Figure 16, the outward-facing surface 600a of the headgear 600 can include, *inter alia,* headgear size indicators 609b, 628, one or more stiffening areas 624a, 624b, 632a, 632b, and a plurality of connection or attachment sections 626a, 626b. The size indicated by the headgear size indicators 609b, 628 can be relative sizing, such as extra-small (XS), small (S), medium (M), large (L), and extra-large (XL). In some configurations, the headgear size indicators 609b, 628 can indicate the range of head circumferences with which the headgear 600 can be suitable for use. For example, as shown in the illustrated example in Figure 16, a large (L) headgear 600 may be suitable for patients with a head circumference size of between about 30 cm to about 36 cm. As another example, a medium (M) headgear may be suitable for patients with a head circumference size of between about 25 cm to about 30 cm. In some configurations, the size indicated by the headgear size indicators 609b, 628 can indicate an absolute size.

The one or more stiffening areas 624a, 624b, 632a, 632b may comprise a stiffening material configured to stiffen the one or more stiffening areas 624a, 624b, 632a, 632b such that these areas 624a, 624b, 632a, 632b can be less flexible and/or less stretchable relative to other portions of the headgear 600. The stiffening material may comprise polypropylene, a hook and loop material, and/or any other suitable material that is stiff relative to other portions of the headgear 600. The stiffening material may be heat pressed, stitched, sewn, or otherwise applied onto a desired region of the top straps 610a, 610b or another part of the headgear 600.

In some configurations, the one or more stiffening areas 624a, 624b, 632a, 632b may include first and second stiffening areas 624a, 624b, and third and fourth stiffening areas 632a, 632b. In some configurations the one or more stiffening areas 624a, 624b, 632a, 632b may include the headgear connector part or connection zone 606, the protruding tab portions 616a, 616b, the first and second attachment zones 618a, 618b, and the plurality of connection or attachment sections 626a, 626b. The one or more stiffening areas 624a, 624b, 632a, 632b may be located anywhere on the headgear 600. For example, a stiffening material may be used at an attachment point 618a, 618b, 622a, 622b, 626a, 626b to increase the strength and sturdiness of the attachment or at another region to limit or prevent movement of that region when the headgear 600 is fitted onto the patient. In the illustrated configuration, the first and second stiffening areas 624a, 624b may be located on the free end of each of the adjustable ear loops 614a, 614b. The first and second stiffening areas 624a, 624b can strengthen the attachment regions 618a, 618b, 622a, 622b and stabilize the adjustable ear loops 614a, 614b.

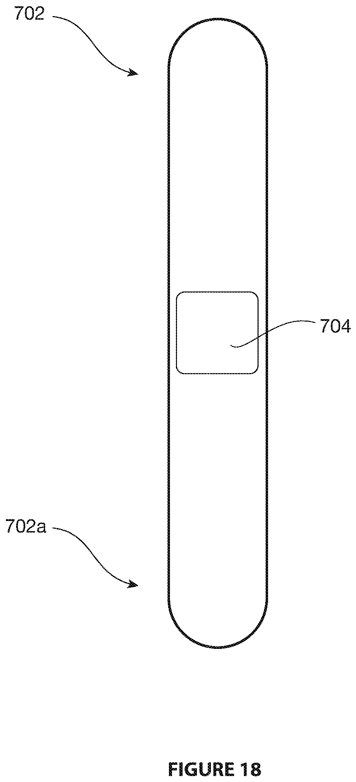

The third and fourth stiffening areas 632a, 632b may be located on respective first and second top straps 610a, 610b. In particular, the third and fourth stiffening areas 632a, 632b may be located on the protruding tab portions 616a, 616b and extend along a portion of the first and second top strap portions 612a, 612b and/or a portion of the backstrap portion 608. The third and fourth stiffening areas 632a, 632b can keep the headgear 600 stable on the patient's head 2 by reducing or limiting rotation that may occur without the stiffening areas. For example, the third and fourth stiffening areas 632a, 632b can reduce or limit rotation of the headgear 600 with or without the forehead member 702, which is further described below in relation to Figures 18-19, attached. In some configurations, the third and fourth stiffening areas 632a, 632b can include respective U-shaped portions 633a, 633b that can extend along respective top strap portions 612a, 612b. The ends of the top strap portions 612a, 612b can be positioned and attached within the U-shaped portions 633a, 633b such that a portion of each U-shape portion 633a, 633b can extend along each side of the ends of the top strap portions 612a, 612b. Other portions of the headgear 600, apart from the one or more stiffening areas 606, 616a, 616b, 618a, 618b, 624a, 624b, 626a, 626b, 632a, 632b can have a degree of stretchiness such that the headgear 600 is suitable to conform and cater to a range of head sizes and shapes.

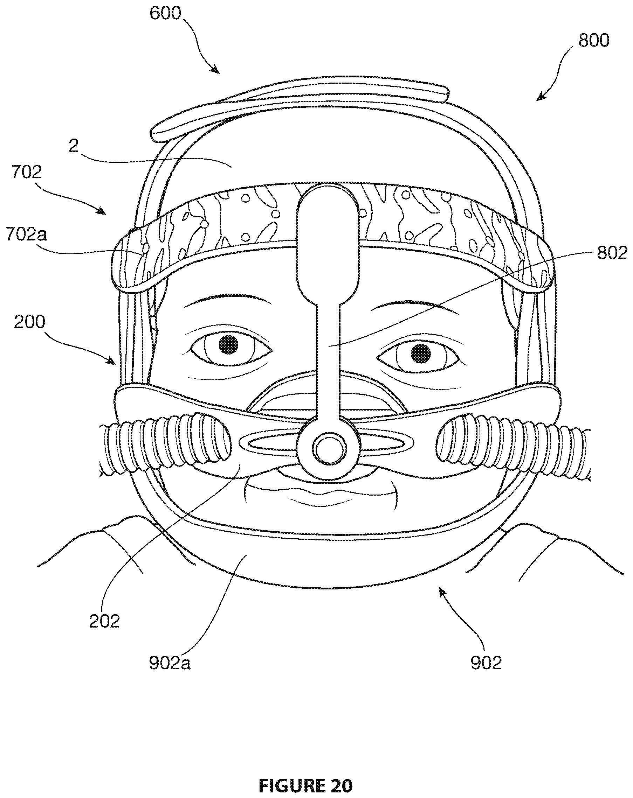

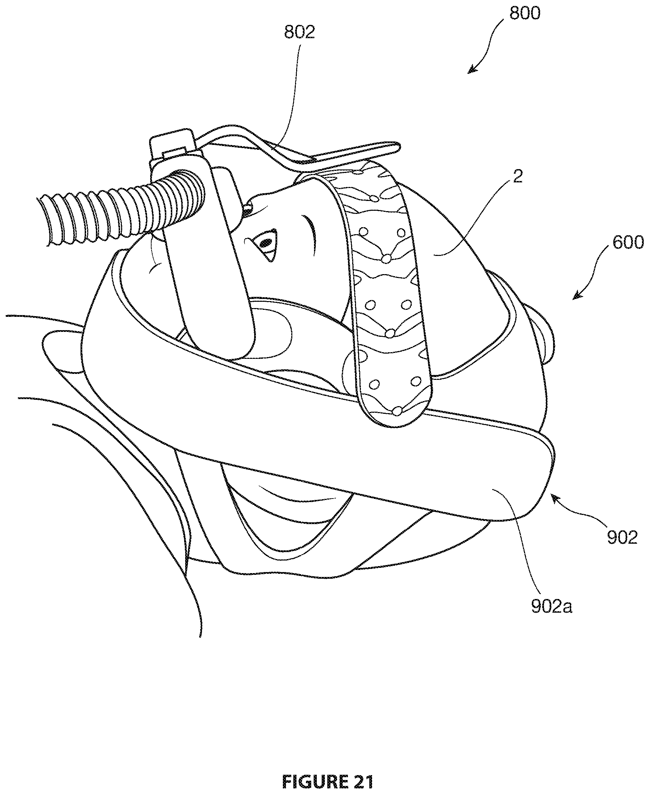

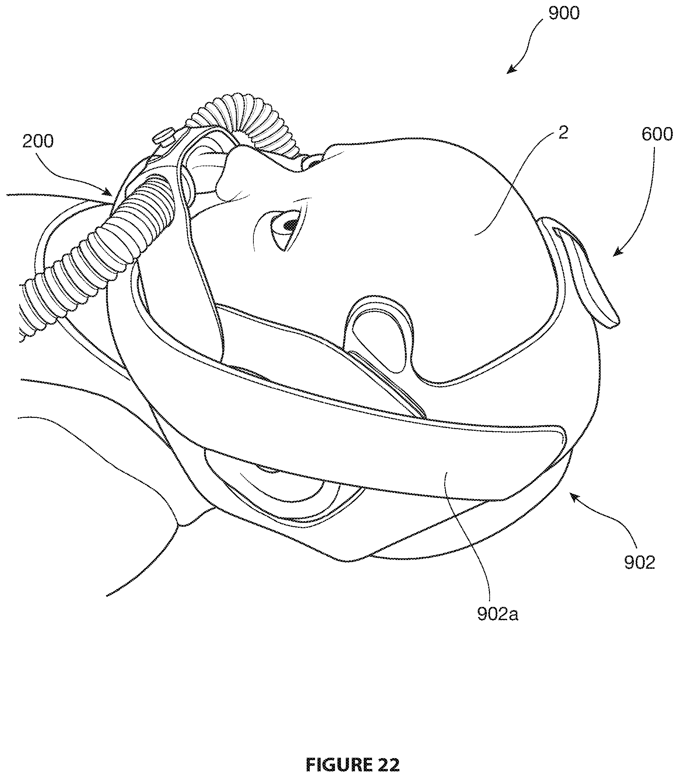

Moreover, the one or more stiffening areas 624a, 624b, 632a, 632b can be configured to cause the adjustable ear loops 614a, 614b to angle inwards (i.e., towards the patient's head 2 in use) when the adjustable ear loops 614a, 614b have been adjusted and attached to the first and second attachment zones 618a, 618b. For example, as shown in Figure 13, an angle 634 between a vertical plane V, which can be substantially parallel or parallel to a side of the patient's head 2, and a portion of the adjustable ear loop 614a, 614b can be between about 20° to about 70°, between about 30° to about 60°, between about 40° to about 50°, or any suitable angle. The one or more stiffening areas 624a, 624b, 632a, 632b can pull the adjustable ear loop 614a, 614b towards the patient's face when the headgear 600 is applied or being applied to the patient such that the headgear 600 can conform easier to the face to allow for simple attachment of the patient interface 200 (shown in Figures 20-22) to the adjustable ear loop 614a, 614b. In some configurations, the first and second stiffening areas 624a, 624b can have a rounded edge at or near the free ends of the adjustable ear loops 614a, 614b and a tapered edge opposite the rounded edge. The tapered edge can be configured to conform to and align with the first and second attachment zones 618a, 618b. In some configurations, such as a large (L) size of the headgear 600, as shown in the illustrated configurations, or other sizes of the headgear 600, additional stiffening material (e.g., polypropylene) can be used at the one or more stiffening areas 624a, 624b, 632a, 632b to achieve this angling effect. In some configurations, the plurality of connection or attachment sections 618a, 618b, 626a, 626b may comprise a hook and loop material that may be sufficient to achieve this angling effect without the use of additional stiffening material (e.g., polypropylene).

As shown in Figure 16, the outward-facing surface 600a of the headgear 600 can include the plurality of connection or attachment sections 626a, 626b. In particular, the plurality of connection or attachment sections 626a, 626b can include a first connection or attachment section 626a and a second connection or attachment section 626b. The first and second connection or attachment sections 626a, 626b can be positioned on any portion of the headgear 600. For example, as show in the illustrated configuration, the first and second connection or attachment sections 626a, 626b can be positioned on the free ends of the adjustable ear loops 614a, 614b. The first and second connection or attachment sections 626a, 626b can include a second part of any of the releasable connection systems described herein. For example, the first and second connection or attachment sections 626a, 626b can be configured to be removably attached to a chin securement member 902, which is further described below in relation to Figures 20-23.

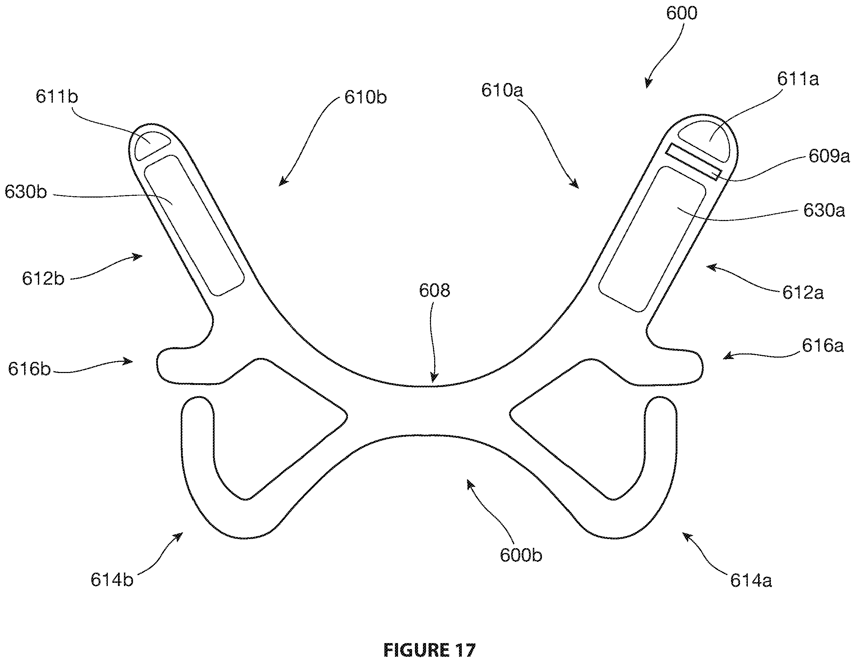

As shown in Figure 17, the patient-contacting surface 600b of the headgear 600 can include, *inter alia,* one or more areas of increased friction 630a, 630b with respect to other portions of the headgear 600. The one or more areas of increased friction 630a, 630b may comprise a tacky material that can increase friction between the patient and the tacky areas 630a, 630b and reduces or minimizes skin damage to the points of contact on the patient's head 2. The tacky material may comprise a tape comprising a polyurethane adhesive film and a polyurethane elastic barrier (e.g., Bemis^{™} tape), neoprene, non-stick silicone, and/or thermoplastic polyurethane. The tacky material may be heat pressed onto a desired region of the top straps 610a, 610b or other parts of the headgear 600. In some configurations, the one or more areas of increased friction 630a, 630b can be configured to stabilize the headgear 600 and the interface 200 (shown in Figures 20-22) in use. In particular, the one or more areas of increased friction 630a, 630b can be positioned at locations where the headgear 600 may move and/or slip relative to the patient's head 2 in use. Advantageously, stabilizing the headgear 600 and the interface 200 can increase patient comfort and maintain a seal with the interface 200 to ensure proper therapy is delivered to the patient.

**In** some configurations, the one or more areas of increased friction 630a, 630b can include a first tacky area 630a and a second tacky area 630b. In the illustrated configuration, the first and second tacky areas 630a, 630b may be located on respective top strap portions 612a, 612b or any desired region on the headgear 600. The first and second tacky areas 630a, 630b may extend along the length of the respective top strap portions 612a, 612b. Advantageously, positioning the first and second tacky areas 630a, 630b on the first and second top strap portions 612a, 612b can limit or substantially prevent the top straps 610a, 610b from sliding forward on the patient's head 2. The first and second tacky areas 630a, 630b can increase friction between the areas 630a, 630b to keep the headgear 600 upright on the patient's head 2.

Another configuration of a removable forehead securement member is illustrated in Figures 18-19. Figures 18-19 show a configuration of a removable forehead securement member 702 that is similar to the removable forehead securement member 302 described with reference to Figures 5-12 and can have similar features. Reference numerals of the same, substantially the same, or corresponding features may share the same last two digits. Any component disclosed in any configuration in this specification can be used in any other configuration.

Figure 18 illustrates the outward-facing surface 702a of the forehead securement member 702. The forehead securement member 702 may come in a variety of sizes to accommodate different head circumferences. In some configurations, the outward- facing surface 702a of the forehead securement member 702 may include a size indicator similar to the headgear size indicator 628. In some configurations, the outward-facing surface 702a of the forehead securement member 702 may be coloured or patterned to indicate the size of the forehead securement member 702 (i.e., different colours or patterns corresponding to different sizes). For example, as shown in Figures 20-21, the outward-facing surface 702a of the forehead securement member 702 may include a pattern or other visual indicator, to indicate a large (L) size. Additionally, or alternatively, other parts of the headgear 100, 600 may be coloured or patterned to indicate a size of the headgear 100, 600. Moreover, similar to the removable forehead securement member 302 discussed above, the outward-facing surface 702a of the forehead securement member 702 can be releasably attachable to removable securement member 802 (Figures 20-21). The removable securement member 802 can be similar to or the same as the removable securement members 402, 502 described with reference to Figures 11-12.

In some configurations, the forehead securement member 702 can include one or more layers. For example, the forehead securement member 702 can include a first layer and a second layer. The first layer can comprise the same or similar material as the body 102, 602 of the headgear 100, 600. The second layer can include a non-stretch material (e.g., the foxpatterned material shown in Figures 20-21) to stabilize the forehead securement member 702 and to reduce stretching of the forehead securement member 702 on the patient's skin, which may otherwise cause damage to the patient's skin.

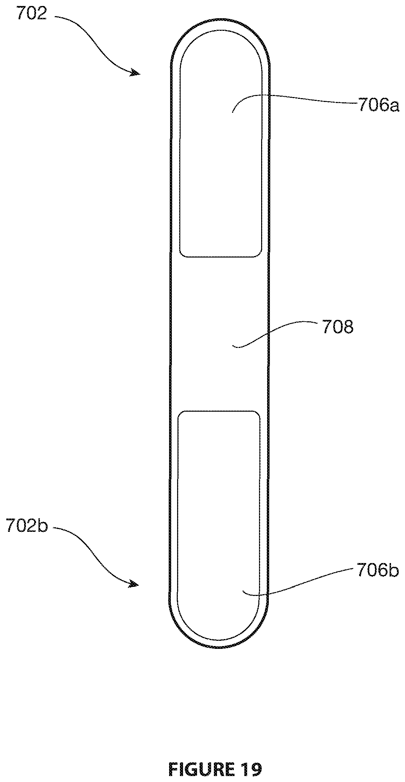

Figure 19 illustrates the patient-contacting surface 702b of the forehead securement member 702. Similar to the removable forehead securement member 302 discussed above, the patient-contacting surface of the removable forehead securement member 702 can include one or more attachment or connection zones 706a, 706b. In particular, the one or more attachment or connection zones 706a, 706b can include a first attachment or connection zone 706a and a second attachment or connection zone 706b. The first and second attachment or connections zones 706a, 706b may be positioned on opposite ends of the forehead securement member 702 or elsewhere on the forehead securement member 702. Similar to the removable forehead securement member 302, the first and second attachment or connection zones 706a, 706b can be configured to removably attach to the third and fourth connection zones 122a, 122b, 622a, 622b on the headgear 100, 600. In some configurations, the forehead securement member 702 may include one or more areas of increased friction 708. In particular, the forehead securement member 702 may include a tacky area 708. The tacky area 708 may be the same as or similar to the first and second tacky areas 630a, 630b of the headgear 600. The tacky area 708 can be configured to maintain and/or stabilize the position of the forehead securement member 702 on the patient's forehead. For example, the tacky area 708 can reduce or prevent the movement of the forehead securement member 702 on the patient's forehead in use. Additionally, or alternatively, the tacky area 708 can be configured to stabilize a removable securement member 402, 502, 802 (shown in Figures 11-12 and 20-21) attached to this securement member 702 such that a seal between the interface 200 and the patient isn't disrupted. The tacky area 708 may be positioned between the first and second connections zones 706a, 706b.

Figures 20-22 illustrate a headgear assembly 800 connected to an interface 200. The headgear assembly 800 can include the headgear 600 and a chin securement member 902 configured to keep the patient's mouth closed such that the therapy (e.g., CPAP therapy) can be delivered effectively to the patient in use. In some configurations, the headgear assembly 800 includes the headgear 100. The chin securement member 902 can comprise a elongate strap that can extend underneath the patient's chin in use. The material and construction of the chin securement member 902 can be the same as or similar to the body 602 of the headgear 600. The chin securement member 902 can be removable from the body 602 or a remainder of the headgear 600; however, in alternative configurations the chin securement member 902 can be integral with or permanently attached to the headgear 600. For example, one end of the chin securement member 902 could be integral with or permanently connected to the headgear 600 and the other end could be releasably connectable to the headgear 600. Alternatively, both ends of the chin securement member 902 could be integral with or permanently connected to the headgear 600.

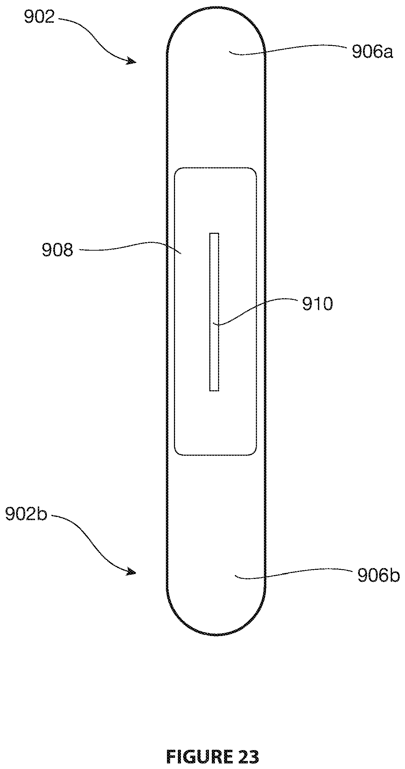

The chin securement member 902 can include a patient-contacting surface 902b (Figure 23) and an outward-facing surface 902a (Figures 20-22) opposite the patient-contacting surface 902b. As shown in Figure 23, the patient-contacting surface 902b of the chin securement member 902 can include one or more connection zones 906a, 906b that can include a first part of a two-part releasable connection system. For example, the one or more connection zones 906a, 906b can include a first connection zone 906a and a second connection zone 906b. The first and second connection zones 906a, 906b can be positioned on opposite ends of the chin securement member 902 or elsewhere on the chin securement member 902. As discussed above, the first and second connection or attachment sections 626a, 626b of the headgear 600 can include a second part of the two-part releasable connection system. For example, the first and second connection zones 906a, 906b of the chin securement member 902 can include a loop material that corresponds with a hook material of first and second connection or attachment sections 626a, 626b such that the first and second connection or attachment sections 626a, 626b are attachable to any portion of the first and second connection zones 906a, 906b of the chin securement member 902. The two-part releasable connection system can comprise any of the releasable connection systems described herein.

As shown in Figure 23, the chin securement member 902 may include one or more areas of increased friction 908. In particular, the chin securement member 902 may include a tacky area 908. The tacky area 908 may be the same as or similar to the first and second tacky areas 630a, 630b of the headgear 600. The tacky area 908 can be configured to maintain and stabilize the position of the chin securement member 902 on the patient's chin in use. The tacky area 908 may be positioned between the first and second connections zones 906a, 906b of the chin securement member 902, or elsewhere on the chin securement member 902. Moreover, the tacky area 908 can prevent this portion of the chin securement member 902 from attaching to the headgear 600. Advantageously, this assists in the even and/or symmetrical application of the chin securement member 902 to the patient and/or the headgear 600. An even and/or symmetrical application of the chin securement member 902 can prevent and/or mitigate an uneven application of force on either side of the chin securement member 902, which can cause instability of the headgear assembly 800, 900 and the interface 200 on the user's head resulting in a potential loss of therapy. Additionally, or alternatively, the chin securement member 902 can include a slit 910 for increased stability of the chin securement member 902 in use. The slit 910 may be positioned on the chin securement member 902 such that the slit 910 aligns with the patient's chin in use. For example, the slit 910 may be positioned approximately in the middle of the chin securement member 902 and/or the tacky area 908.

### Terminology

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to". Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

The term "plurality" refers to two or more of an item. Recitations of quantities, dimensions, sizes, formulations, parameters, shapes and other characteristics should be construed as if the term "about" or "approximately" precedes the quantity, dimension, size, formulation, parameter, shape or other characteristic. The terms "about" or "approximately" mean that quantities, dimensions, sizes, formulations, parameters, shapes and other characteristics need not be exact, but may be approximated and/or larger or smaller, as desired, reflecting acceptable tolerances, conversion factors, rounding off, measurement error and the like and other factors known to those of skill in the art. Recitations of quantities, dimensions, sizes, formulations, parameters, shapes and other characteristics should also be construed as if the term "substantially" precedes the quantity, dimension, size, formulation, parameter, shape or other characteristic. The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes, or tends toward, a particular value, amount, or characteristic. For example, as the context may dictate, the term "generally linear" can mean something that departs from exactly parallel by less than or equal to 15°.

Numerical data may be expressed or presented herein in a range format. It is to be understood that such a range format is used merely for convenience and brevity and thus should be interpreted flexibly to include not only the numerical values explicitly recited as the limits of the range, but also interpreted to include all of the individual numerical values or sub-ranges encompassed within that range as if each numerical value and sub-range is explicitly recited. As an illustration, a numerical range of" 1 to 5" should be interpreted to include not only the explicitly recited values of about 1 to about 5, but should also be interpreted to also include individual values and sub-ranges within the indicated range. Thus, included in this numerical range are individual values such as 2, 3 and 4 and sub-ranges such as "1 to 3," "2 to 4" and "3 to 5," etc. This same principle applies to ranges reciting only one numerical value (e.g., "greater than 1") and should apply regardless of the breadth of the range or the characteristics being described.

A plurality of items may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. Furthermore, where the terms "and" and "or" are used in conjunction with a list of items, they are to be interpreted broadly, in that any one or more of the listed items may be used alone or in combination with other listed items. The term "alternatively" refers to selection of one of two or more alternatives, and is not intended to limit the selection to only those listed alternatives or to only one of the listed alternatives at a time, unless the context clearly indicates otherwise.

Reference to any prior art in this specification is not, and should not be taken as, an acknowledgement or any form of suggestion that that prior art forms part of the common general knowledge in the field of endeavor in any country in the world. Furthermore, any references to other patent specifications, other external documents, or other sources of information are generally for the purpose of providing a context for discussing the features of certain embodiments. Unless specifically stated otherwise, reference to such external documents is not to be construed as an admission that such documents, or such sources of information, in any jurisdiction, are prior art, or form part of the common general knowledge in the art.

## Claims

1. A headgear (100, 600) for use with a respiratory patient interface (200), the headgear comprising:
a backstrap portion (108, 608);
at least one top strap portion (110, 110a, 110b, 610a, 610b);
a first adjustable ear loop (114a, 614a) extending from a first end of the backstrap portion (108, 608), the first adjustable ear loop comprising a first connection portion (626a) configured to be removably attachable to a first attachment zone (118a, 618a) of the headgear such that the first adjustable ear loop (114a, 614a) defines a first ear opening (120a), wherein the first attachment zone is configured to be attachable to different portions along the first connection portion to adjust a size of the first ear opening; and
a second adjustable ear loop (114b, 614b) extending from a second end of the backstrap portion, the second adjustable ear loop comprising a second connection portion (626b) configured to be removably attachable to a second attachment zone (118b, 618b) of the headgear such that the second adjustable ear loop defines a second ear opening (120b), wherein the second attachment zone is configured to be attachable to different portions along the second connection portion to adjust a size of the second ear opening.

2. The headgear of Claim 1, wherein the first adjustable ear loop (114a, 614a) follows a path defining a first ear opening (120a) and the second adjustable ear loop (114b, 614b) follows a path defining a second ear opening (120b), and
wherein the first ear opening (120a) and the second ear opening (120b) comprises a rounded triangular shape.

3. The headgear of Claim 1 or 2, wherein the first and second attachment zones (118a, 618a, 118b, 618b) comprise one of a hook material or a loop material, wherein the first and second connection portions comprise the other of the hook material or the loop material.

4. The headgear of any one of Claims 1 to 3, wherein the at least one top strap portion (110, 110a, 110b, 610a, 610b) comprises a first top strap portion (112a, 612a) and a second top strap portion (112b, 612b) that is selectively connectable to the first top strap portion.

5. The headgear of Claim 4, wherein the first top strap portion (112a, 612a) extends from a first end of the backstrap portion and the second top strap portion (112b, 612b) extends from a second end of the backstrap portion (108, 608).

6. The headgear of Claim 4 or 5, wherein the first attachment zone (118a, 618a) is located on the first top strap portion (112a, 612a) and/or a first protruding tab (116a, 616a) of the first top strap portion, and the second attachment zone (118b, 618b) is located on the second top strap portion (112b, 612b) and/or a second protruding tab (116b, 616b) of the second top strap portion.

7. The headgear of any one of Claims 4 to 6, wherein the first top strap portion (112a, 612a) comprises a first protruding portion (116a, 616a) and the second top strap portion (112b, 612b) comprises a second protruding portion (116b, 616b).

8. The headgear of Claim 7, wherein the first protruding portion (116a, 616a) comprises a third attachment zone (122a, 622a) and the second protruding portion (116b, 616b) comprises a fourth attachment zone (122b, 622b).

9. The headgear of Claim 8, further comprising a forehead securement (302, 702) member configured to be removably attachable to the third and fourth attachment zones (122a, 622a, 122b, 622b).

10. The headgear of any one of Claims 4 to 9, wherein the first and second top strap portions (110a, 110b, 610a, 610b) are configured to be adjustable to conform to a size of the head of the user.

11. The headgear of any one of Claims 4 to 10, wherein the first top strap portion (112a, 612a) comprises a slot (109a, 609a) configured to receive the second top strap portion (112b, 612b).

12. The headgear of any one of Claims 1 to 11, wherein the first and second adjustable ear loops (114a, 114b, 614a, 614b) are symmetrical about the backstrap portion (108, 608).

13. The headgear of any one of Claims 1 to 12, wherein the first and second adjustable ear loops (114a, 114b, 614a, 614b) comprise respective ear loop size indicators (115a, 115b), the respective ear loop size indicators correspond to a respective size of the first and second ear openings.

14. The headgear of any one of Claims 1 to 13, wherein the first and second adjustable ear loops (114a, 114b, 614a, 614b) comprise respective attachment zones (118a, 118b, 618a, 618b).

15. The headgear of Claim 14, wherein the respective attachment zones (118a, 118b, 618a, 618b) are configured to be removably attachable to the respiratory patient interface (200).

## Patentansprüche

1. Eine Kopfvorrichtung (100, 600) zur Verwendung mit einer Atmungspatientenschnittstelle (200), wobei die Kopfvorrichtung Folgendes beinhaltet:
einen Hinterriemenabschnitt (108, 608);
mindestens einen Oberriemenabschnitt (110, 110a, 110b, 610a, 610b);
eine erste anpassbare Ohrschlaufe (114a, 614a), die sich von einem ersten Ende des Hinterriemenabschnitts (108, 608) erstreckt, wobei die erste anpassbare Ohrschlaufe einen ersten Verbindungsabschnitt (626a) beinhaltet, der konfiguriert ist, um entfernbar an einer ersten Befestigungszone (118a, 618a) der Kopfvorrichtung befestigt werden zu können, sodass die erste anpassbare Ohrschlaufe (114a, 614a) eine erste Ohröffnung (120a) definiert, wobei die erste Befestigungszone konfiguriert ist, um an verschiedenen Abschnitten entlang des ersten Verbindungsabschnitts befestigt werden zu können, um eine Größe der ersten Ohröffnung anzupassen; und
eine zweite anpassbare Ohrschlaufe (114b, 614b), die sich von einem zweiten Ende des Hinterriemenabschnitts erstreckt, wobei die zweite anpassbare Ohrschlaufe einen zweiten Verbindungsabschnitt (626b) beinhaltet, der konfiguriert ist, um entfernbar an einer zweiten Befestigungszone (118b, 618b) der Kopfvorrichtung befestigt werden zu können, sodass die zweite anpassbare Ohrschlaufe eine zweite Ohröffnung (120b) definiert, wobei die zweite Befestigungszone konfiguriert ist, um an verschiedenen Abschnitten entlang des zweiten Verbindungsabschnitts befestigt werden zu können, um eine Größe der zweiten Ohröffnung anzupassen.

2. Kopfvorrichtung gemäß Anspruch 1, wobei die erste anpassbare Ohrschlaufe (114a, 614a) einem Pfad folgt, der eine erste Ohröffnung (120a) definiert, und die zweite anpassbare Ohrschlaufe (114b, 614b) einem Pfad folgt, der eine zweite Ohröffnung (120b) definiert, und
wobei die erste Ohröffnung (120a) und die zweite Ohröffnung (120b) eine abgerundete dreieckige Form beinhalten.

3. Kopfvorrichtung gemäß Anspruch 1 oder 2, wobei die erste und die zweite Befestigungszone (118a, 618a, 118b, 618b) eines von einem Hakenmaterial oder einem Schlaufenmaterial beinhalten, wobei der erste und der zweite Verbindungsabschnitt das andere von dem Hakenmaterial oder dem Schlaufenmaterial beinhalten.

4. Kopfvorrichtung gemäß einem der Ansprüche 1 bis 3, wobei der mindestens eine Oberriemenabschnitt (110, 110a, 110b, 610a, 610b) einen ersten Oberriemenabschnitt (112a, 612a) und einen zweiten Oberriemenabschnitt (112b, 612b), der selektiv mit dem ersten Oberriemenabschnitt verbindbar ist, beinhaltet.

5. Kopfvorrichtung gemäß Anspruch 4, wobei sich der erste Oberriemenabschnitt (112a, 612a) von einem ersten Ende des Hinterriemenabschnitts erstreckt und sich der zweite Oberriemenabschnitt (112b, 612b) von einem zweiten Ende des Hinterriemenabschnitts (108, 608) erstreckt.

6. Kopfvorrichtung gemäß Anspruch 4 oder 5, wobei sich die erste Befestigungszone (118a, 618a) an dem ersten Oberriemenabschnitt (112a, 612a) und/oder einer ersten vorstehenden Lasche (116a, 616a) des ersten Oberriemenabschnitts befindet und sich die zweite Befestigungszone (118b, 618b) an dem zweiten Oberriemenabschnitt (112b, 612b) und/oder einer zweiten vorstehenden Lasche (116b, 616b) des zweiten Oberriemenabschnitts befindet.

7. Kopfvorrichtung gemäß einem der Ansprüche 4 bis 6, wobei der erste Oberriemenabschnitt (112a, 612a) einen ersten vorstehenden Abschnitt (116a, 616a) beinhaltet und der zweite Oberriemenabschnitt (112b, 612b) einen zweiten vorstehenden Abschnitt (116b, 616b) beinhaltet.

8. Kopfvorrichtung gemäß Anspruch 7, wobei der erste vorstehende Abschnitt (116a, 616a) eine dritte Befestigungszone (122a, 622a) beinhaltet und der zweite vorstehende Abschnitt (116b, 616b) eine vierte Befestigungszone (122b, 622b) beinhaltet.

9. Kopfvorrichtung gemäß Anspruch 8, die ferner ein Stirnsicherungselement (302, 702) beinhaltet, das konfiguriert ist, um entfernbar an der dritten und der vierten Befestigungszone (122a, 622a, 122b, 622b) befestigt werden zu können.

10. Kopfvorrichtung gemäß einem der Ansprüche 4 bis 9, wobei der erste und der zweite Oberriemenabschnitt (110a, 110b, 610a, 610b) konfiguriert sind, um anpassbar zu sein, um mit einer Größe des Kopfes des Benutzers übereinzustimmen.

11. Kopfvorrichtung gemäß einem der Ansprüche 4 bis 10, wobei der erste Oberriemenabschnitt (112a, 612a) einen Schlitz (109a, 609a) beinhaltet, der konfiguriert ist, um den zweiten Oberriemenabschnitt (112b, 612b) aufzunehmen.

12. Kopfvorrichtung gemäß einem der Ansprüche 1 bis 11, wobei die erste und die zweite anpassbare Ohrschlaufe (114a, 114b, 614a, 614b) um den Hinterriemenabschnitt (108, 608) symmetrisch sind.

13. Kopfvorrichtung gemäß einem der Ansprüche 1 bis 12, wobei die erste und die zweite anpassbare Ohrschlaufe (114a, 114b, 614a, 614b) jeweilige Ohrschlaufengrößenindikatoren (115a, 115b) beinhalten, wobei die jeweiligen Ohrschlaufengrößenindikatoren einer jeweiligen Größe der ersten und der zweiten Ohröffnung entsprechen.

14. Kopfvorrichtung gemäß einem der Ansprüche 1 bis 13, wobei die erste und die zweite anpassbare Ohrschlaufe (114a, 114b, 614a, 614b) jeweilige Befestigungszonen (118a, 118b, 618a, 618b) beinhalten.

15. Kopfvorrichtung gemäß Anspruch 14, wobei die jeweiligen Befestigungszonen (118a, 118b, 618a, 618b) konfiguriert sind, um entfernbar an der Atmungspatientenschnittstelle (200) befestigt werden zu können.

## Revendications

1. Un harnais (100, 600) destiné à être utilisé avec une interface respiratoire de patient (200), le harnais comprenant :
une portion sangle arrière (108, 608) ;
au moins une portion sangle supérieure (110, 110a, 110b, 610a, 610b) ;
une première boucle auriculaire réglable (114a, 614a) s'étendant à partir d'une première extrémité de la portion sangle arrière (108, 608), la première boucle auriculaire réglable comprenant une première portion de liaison (626a) configurée pour être attachable de manière amovible à une première zone d'attache (118a, 618a) du harnais de telle sorte que la première boucle auriculaire réglable (114a, 614a) définit une première ouverture auriculaire (120a), où la première zone d'attache est configurée pour être attachable à différentes portions le long de la première portion de liaison afin de régler une taille de la première ouverture auriculaire ; et
une deuxième boucle auriculaire réglable (114b, 614b) s'étendant à partir d'une deuxième extrémité de la portion sangle arrière, la deuxième boucle auriculaire réglable comprenant une deuxième portion de liaison (626b) configurée pour être attachable de manière amovible à une deuxième zone d'attache (118b, 618b) du harnais de telle sorte que la deuxième boucle auriculaire réglable définit une deuxième ouverture auriculaire (120b), où la deuxième zone d'attache est configurée pour être attachable à différentes portions le long de la deuxième portion de liaison afin de régler une taille de la deuxième ouverture auriculaire.

2. Le harnais de la revendication 1, où la première boucle auriculaire réglable (114a, 614a) suit un chemin définissant une première ouverture auriculaire (120a) et la deuxième boucle auriculaire réglable (114b, 614b) suit un chemin définissant une deuxième ouverture auriculaire (120b), et
où la première ouverture auriculaire (120a) et la deuxième ouverture auriculaire (120b) comprennent une forme triangulaire arrondie.

3. Le harnais de la revendication 1 ou de la revendication 2, où les première et deuxième zones d'attache (118a, 618a, 118b, 618b) comprennent l'un d'un matériau à crochet ou d'un matériau à boucle, où les première et deuxième portions de liaison comprennent l'autre du matériau à crochet ou du matériau à boucle.

4. Le harnais de l'une quelconque des revendications 1 à 3, où l'au moins une portion sangle supérieure (110, 110a, 110b, 610a, 610b) comprend une première portion sangle supérieure (112a, 612a) et une deuxième portion sangle supérieure (112b, 612b) qui est reliable sélectivement à la première portion sangle supérieure.

5. Le harnais de la revendication 4, où la première portion sangle supérieure (112a, 612a) s'étend à partir d'une première extrémité de la portion sangle arrière et la deuxième portion sangle supérieure (112b, 612b) s'étend à partir d'une deuxième extrémité de la portion sangle arrière (108, 608).

6. Le harnais de la revendication 4 ou de la revendication 5, où la première zone d'attache (118a, 618a) est située sur la première portion sangle supérieure (112a, 612a) et/ou une première languette en saillie (116a, 616a) de la première portion sangle supérieure, et la deuxième zone d'attache (118b, 618b) est située sur la deuxième portion sangle supérieure (112b, 612b) et/ou une deuxième languette en saillie (116b, 616b) de la deuxième portion sangle supérieure.

7. Le harnais de l'une quelconque des revendications 4 à 6, où la première portion sangle supérieure (112a, 612a) comprend une première portion en saillie (116a, 616a) et la deuxième portion sangle supérieure (112b, 612b) comprend une deuxième portion en saillie (116b, 616b).

8. Le harnais de la revendication 7, où la première portion en saillie (116a, 616a) comprend une troisième zone d'attache (122a, 622a) et la deuxième portion en saillie (116b, 616b) comprend une quatrième zone d'attache (122b, 622b).

9. Le harnais de la revendication 8, comprenant en outre un élément de fixation au front (302, 702) configuré pour être attachable de manière amovible aux troisième et quatrième zones d'attache (122a, 622a, 122b, 622b).

10. Le harnais de l'une quelconque des revendications 4 à 9, où les première et deuxième portions sangles supérieures (110a, 110b, 610a, 610b) sont configurées pour être réglables de manière à épouser une taille de la tête de l'utilisateur.

11. Le harnais de l'une quelconque des revendications 4 à 10, où la première portion sangle supérieure (112a, 612a) comprend une fente (109a, 609a) configurée pour recevoir la deuxième portion sangle supérieure (112b, 612b).

12. Le harnais de l'une quelconque des revendications 1 à 11, où les première et deuxième boucles auriculaires réglables (114a, 114b, 614a, 614b) sont symétriques autour de la portion sangle arrière (108, 608).

13. Le harnais de l'une quelconque des revendications 1 à 12, où les première et deuxième boucles auriculaires réglables (114a, 114b, 614a, 614b) comprennent des indicateurs de taille de boucle auriculaire respectifs (115a, 115b), les indicateurs de taille de boucle auriculaire respectifs correspondant à une taille respective des première et deuxième ouvertures auriculaires.

14. Le harnais de l'une quelconque des revendications 1 à 13, où les première et deuxième boucles auriculaires réglables (114a, 114b, 614a, 614b) comprennent des zones d'attache respectives (118a, 118b, 618a, 618b).

15. Le harnais de la revendication 14, où les zones d'attache respectives (118a, 118b, 618a, 618b) sont configurées pour être attachables de manière amovible à l'interface respiratoire de patient (200).
